# EUROPÄISCHE PATENTSCHRIFT

(11) **EP 1 421 080 B1**
(45) Veröffentlichungstag und Bekanntmachung des Hinweises auf die Patenterteilung: **09.02.2005**
(21) Anmeldenummer: 02764808.8
(22) Anmeldetag: 30.07.2002
(51) Int. Cl.: C07D 409/14, C07D 409/12, A01N 43/54, A01N 43/707, A01N 43/713

(54) **N-HETEROCYCLYLSUBSTITUIERTE THIENYLOXY-PYRIMIDINE ALS HERBIZIDE**
N-HETEROCYCLYL SUBSTITUTED THIENYLOXY-PYRIMIDINES USED AS HERBICIDES
THIENYLOXY-PYRIMIDINES SUBSTITUEES PAR N-HETEROCYCLYLE S'UTILISANT COMME HERBICIDES

(30) Priorität: 17.08.2001 DE 10139404
(43) Veröffentlichungstag der Anmeldung: 26.05.2004
(73) Patentinhaber: BASF AKTIENGESELLSCHAFT, 67056 Ludwigshafen (DE)
(72) Erfinder: PARRA RAPADO, Liliana, 77654 Offenburg (DE); VON DEYN, Wolfgang, 67435 Neustadt (DE); HOFMANN, Michael, 67059 Ludwigshafen (DE); BAUMANN, Ernst, 67373 Dudenhofen (DE); KORDES, Markus, 67227 Frankenthal (DE); MISSLITZ, Ulf, 67433 Neustadt (DE); ZAGAR, Cyrill, 68167 Mannheim (DE); WITSCHEL, Matthias, 67098 Bad Dürkheim (DE); LANDES, Andreas, 67354 Römerberg (DE)
(86) Internationale Anmeldenummer: PCT/EP2002/008451
(87) Internationale Veröffentlichungsnummer: WO 2003/016308

(56) Entgegenhaltungen:
- EP-A- 0 819 690
- EP-A- 0 902 026
- WO-A-00/71536
- WO-A-01/97613
- WO-A-98/40379
- WO-A-99/24427
- US-A- 6 121 202

## Beschreibung

Die vorliegende Erfindung betrifft N-heterocyclylsubstituierte Thienyloxy-Pyrimidine der Formel I 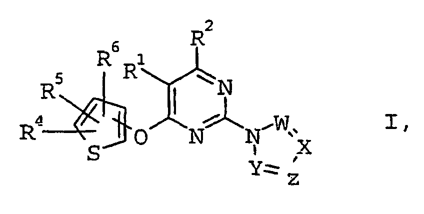 in der die Variablen die folgenden Bedeutungen haben:
- W, X, Y, Z: unabhängig voneinander N oder CR³, wobei mindestens eine der Variablen CR³ bedeutet;
- R¹: Wasserstoff, Halogen, Cyano, C₁-C₆-Alkyl, C₁-C₆-Halogenalkyl, C₁-C₆-Alkoxy oder C₁-C₆-Halogenalkoxy;
- _{R}2: Wasserstoff, Halogen, Cyano, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₆-Alkoxy, C₃-C₆-Alkenyloxy, C₃-C₆-Alkinyloxy, C₁-C₆-Halogenalkoxy, C₁-C₆-Alkoxy-C₁-C₄-alkyl, C₁-C₆-Alkylamino, Di-(C₁-C₆-alkyl)amino, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, COOR⁷ oder CONR⁸R⁹;
- R³: Wasserstoff, Halogen, Cyano, Nitro, C₁-C₆-Alkyl, C₁-C₆-Halogenalkyl, C₁-C₆-Alkoxy, C₁-C₆-Halogenalkoxy, C₁-C₆-Alklylthio, C₁-C₆-Halogenalkylthio, C₁-C₆-Alkylsulfonyl oder COOR⁷;
- R⁴, R⁵, R⁶: Wasserstoff, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfonyl oder C₁-C₆-Halogenalkylsulfonyl;

- R⁷: Wasserstoff, C₁-C₄-Alkyl, C₃-C₄-Alkenyl, C₃-C₄-Alkinyl oder C₁-C₄-Halogenalkyl;
- R⁸: Wasserstoff, C₁-C₄-Alkyl, C₃-C₄-Alkenyl, C₃-C₄-Alkinyl oder C₁-C₄-Alkoxy;
- R⁹: Wasserstoff, C₁-C₄-Alkyl, C₃-C₄-Alkenyl oder C₃-C₄-Alkinyl;
sowie deren landwirtschaftlich brauchbaren Salze.

Außerdem betrifft die Erfindung Zwischenprodukte und Verfahren zur Herstellung von Verbindungen der Formel I, Mittel, welche diese enthalten, sowie die Verwendung dieser Derivate oder der diese Derivate enthaltende Mittel zur Schadpflanzenbekämpfung.

In WO 98/40379 werden Heteroaryl-Azol-Herbizide beschrieben. Aus WO 99/24427 sind herbizid wirkende Furanyl- und Thienyloxyazine bekannt.

Die herbiziden Eigenschaften der bisher bekannten Verbindungen bzw. die Verträglichkeiten gegenüber Kulturpflanzen können jedoch nur bedingt befriedigen. Es lag daher dieser Erfindung die Aufgabe zugrunde, insbesondere herbizid wirksame Verbindungen mit verbesserten Eigenschaften zu finden.

Demgemäß wurden die N-heterocyclylsubstituierten Thienyloxy-Pyrimidine der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von :brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R⁹ genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Alkoxy-, Halogenalkoxy-,Alkylthio-, Halogenalkylthio-, Alkylsulfinyl-, Halogenalkylsulfinyl-, Alkylsulfonyl-, Halogenalkylsulfonyl-, Alkenyloxy-, Alkinyloxy-, Alkylamino- und Dialkylamino-Teile können geradkettig oder verzweigt sein. Sofern nicht anders angegeben tragen halogenierte Substituenten vorzugsweise ein bis fünf, insbesondere ein bis drei, gleiche oder verschiedene Halogenatome. Die Bedeutung Halogen steht jeweils für Fluor, Chlor, Brom oder Iod.

Ferner bedeuten beispielsweise:
◆ C₁-C₄-Alkyl: sowie die Alkylteile von Hydroxy-C₁-C₄-alkyl, Hydroxy-C₁-C₄-alkoxy-C₁-C₄-alkyl, Tri(C₁-C₄-alkyl)sulfonium und Tri(C₁-C₄-alkyl)sulfoxonium: z.B. Methyl, Ethyl, Propyl, 1-Methylethyl, Butyl, 1-Methylpropyl, 2-Methylpropyl und 1,1-Dimethylethyl;
◆ C₁-C₆-Alkyl: C₁-C₄-Alkyl, wie voranstehend genannt, sowie z.B. Pentyl, 1-Methylbutyl, 2-Methylbutyl, 3-Methylbutyl, 2,2-Dimethylpropyl, 1-Ethylpropyl, Hexyl, 1.1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
◆ C₂-C₆-Alkenyl: z.B. Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
◆ C₂-C₆-Alkinyl: z.B. Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
◆ C₁-C₄-Halogenalkyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
◆ C₁-C₆-Halogenalkyl: C₁-C₄-Halogenalkyl wie voranstehend genannt, sowie z.B. 5-Fluorpentyl, 5-Chlorpentyl, 5-Brompenuyl, 5-Iodpentyl, Undecafluorpentyl, 6-Fluorhexyl, 6-Chlorhexyl, 6-Bromhexyl, 6-Iodhexyl und Dodecafluorhexyl;
◆ C₂-C₆-Halogenalkenyl: ein C₂-C₆-Alkenylrest, wie voranstehend genannt, der partiell oder vollständig durch Fluor, Chlor, Brom und/oder Iod substituiert ist, z.B. 2-Chlorvinyl, 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromvinyl, 2-Bromallyl, 3-Bromallyl, 2,3-Dibromallyl, 3,3-Dibromallyl, 2,3,3-Tribromallyl oder 2,3-Dibrombut-2-enyl;
◆ C₂-C₆-Halogenalkinyl: ein C₂-C₆-Alkinylrest, wie voranstehend genannt, der partiell oder vollständig durch Fluor, Chlor, Brom und/oder Iod substituiert ist, z.B. 1,1-Difluorprop-2-in-1-yl, 3-Iod-prop-2-in-1-yl, 4-Fluorbut-2-in-1-yl, 4-Chlorbut-2in-1-yl, 1,1-Difluorbut-2-in-1-yl, 4-Iodbut-3-in-1-yl, 5-Fluorpent-3-in-1-yl, 5-Iodpent-4-in-1-yl, 6-Fluorhex-4-in-1-yl oder 6-Iodhex-5-in-1-yl;
◆ C₁-C₄-Alkoxy: sowie die Alkoxyteile von Hydroxy-C₁-C₄-alkoxy-C₁-C₄-alkyl, z.B. Methoxy, Ethoxy, Propoxy, 1-Methylethoxy, Butoxy, 1-Methylpropoxy, 2-Methylpropoxy und 1,1-Dimethylethoxy;
◆ C₁-C₆-Alkoxy: C₁-C₄-Alkoxy wie voranstehend genannt, sowie z.B. Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy,1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
◆ C₃-C₆-Alkenyloxy: z.B. Prop-1-en-1-yloxy, Prop-2-en-1-yloxy, 1-Methylethenyloxy, Buten-1-yloxy, Buten-2-yloxy, Buten-3-yloxy, 1-Methyl-prop-1-en-1-yloxy, 2-Methyl-prop-1-en-1-yloxy, 1-Methyl-prop-2-en-1-yloxy, 2-Methyl-prop-2-en-1-yloxy, Penten-1-yloxy, Penten-2-yloxy, Penten-3-yloxy, Penten-4-yloxy, 1-Methyl-but-1-en-1-yloxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methyl-but-2-en-1-yloxy, 1-Methylbut-3-en-1-yloxy, 2-Methyl-but-3-en-1-yloxy, 3-Methylbut-3-en-1-yloxy, 1,1-Dimethyl-prop-2-en-1-yloxy, 1,2-Dimethyl-prop-1-en-1-yloxy, 1,2-Dimethyl-prop-2-en-1-yloxy, 1-Ethyl-prop-1-en-2-yloxy, 1-Ethyl-prop-2-en-1-yloxy, Hex-1-en-1-yloxy, Hex-2-en-1-yloxy, Hex-3-en-1-yloxy, Hex-4-en-1-yloxy, Hex-5-en-1-yloxy, 1-Methyl-pent-1-en-1-yloxy, 2-Methyl-pent-1-en-1-yloxy, 3-Methyl-pent-1-en-1-yloxy, 4-Methyl-pent-1-en-1-yloxy, 1-Methyl-pent-2-en-1-yloxy, 2-Methyl-pent-2-en-1-yloxy, 3-Methyl-pent-2-en-1-yloxy, 4-Methylpent-2-en-1-yloxy, 1-Methyl-pent-3-en-1-yloxy, 2-Methylpent-3-en-1-yloxy, 3-Methyl-pent-3-en-1-yloxy, 4-Methylpent-3-en-1-yloxy, 1-Methyl-pent-4-en-1-yloxy, 2-Methylpent-4-en-1-yloxy, 3-Methyl-pent-4-en-1-yloxy, 4-Methylpent-4-en-1-yloxy, 1,1-Dimethyl-but-2-en-1-yloxy, 1,1-Dimethyl-but-3-en-1-yloxy, 1,2-Dimethyl-but-1-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 2,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, 1-Ethyl-but-2-en-1-yloxy, 1-Ethyl-but-3-en-1-yloxy, 2-Ethylbut-1-en-1-yloxy, 2-Ethyl-but-2-en-1-yloxy, 2-Ethylbut-3-en-1-yloxy, 1,1,2-Trimethyl-prop-2-en-1-yloxy, 1-Ethyl-1-methyl-prop-2-en-1-yloxy, 1-Ethyl-2-methylprop-1-en-1-yloxy und 1-Ethyl-2-methyl-prop-2-en-1-yloxy;
◆ C₃-C₆-Alkinyloxy: z.B. Prop-1-in-1-yloxy, Prop-2-in-l-yloxy, But-1-in-1-yloxy, But-1-in-3-yloxy, But-1-in-4-yloxy, But-2-in-1-yloxy, Pent-1-in-1-yloxy, Pent-1-in-3-yloxy, Pent-1-in-4-yloxy, Pent-1-in-5-yloxy, Pent-2-in-1-yloxy, Pent-2-in-4-yloxy, Pent-2-in-5-yloxy, 3-Methyl-but-1-in-3-ylowy, 3-Methyl-but-1-in-4-yloxy, Hex-1-in-1-yloxy, Hex-1-in-3-yloxy, Hex-1-in-4-ylowy, Hex-1-in-5-yloxy, Hex-1-in-6-yloxy, Hex-2-in-1-yloxy, Hex-2-in-4-yloxy, Hex-2-in-5-yloxy, Hex-2-in-6-yloxy, Hex-3-in-1-yloxy, Hex-3-in-2-yloxy, 3-Methylpent-1-in-1-yloxy, 3-Methylpent-1-in-3-yloxy, 3-Methyl-pent-1-in-4-yloxy, 3-Methylpent-1-in-5-yloxy, 4-Methyl-pent-1-in-1-yloxy, 4-Methylpent-2-in-4-yloxy und 4-Methylpent-2-in-5-yloxy;
◆ C₁-C₆-Halogenalkoxy: einen C₁-C₆-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy, Nonafluorbutoxy, 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy und Dodecafluorhexoxy;
◆ C₁-C₆-Alkoxy-C₁-C₄-alkyl: durch C₁-C₆-Alkoxy wie vorstehend genannt substituiertes C₁-C₄-Alkyl, also z.B. für Methoxymethyl, Ethoxymethyl, Propoxymethyl, (1-Methylethoxy)methyl, Butoxymethyl, (1-Methylpropoxy)methyl, (2-Methylpropoxy)methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)ethyl, 2-(2-Methylpropoxy)ethyl, 2-(1,1-Dimethylethoxy)ethyl, 2-(Methoxy)-propyl, 2-(Ethoxy)propyl, 2-(Propoxy)propyl, 2-(1-Methylethoxy)propyl, 2-(Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)propyl, 3-(Butoxy)propyl, 3-(1-Methylpropoxy)propyl, 3-(2-Methylpropoxy)propyl, 3-(1,1-Dimethylethoxy)propyl, 2-(Methoxy)-butyl, 2-(Ethoxy)butyl, 2-(Propoxy)butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)-butyl, 3-(Propowy)butyl, 3-(1-Methylethoxy)butyl, 3-(Butoxy)-butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(Propoxy)butyl, 4-(1-Methylethoxy)butyl, 4-(Butoxy)butyl, 4-(1-Methylpropoxy)butyl, 4-(2-Methylpropoxy)butyl und 4-(1,1-Dimethylethoxy)butyl;
◆ C₁-C₆-Alkylamino:z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3.Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino oder 1-Ethyl-2-methylpropylamino;
◆ Di-(C₁-C₄-alkyl)-amino: z.B. N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N- (2-methylpropyl) amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl) amino, N-(1-Methylethyl)-N-(1-methylpropyl) amino, N-(1-Methylethyl)-N-(2-methylpropyl)amino, N-(1,1-Dimethylethyl)-N-(1-methylethyl) amino, N-Butyl-N-(1-methylpropyl) amino, N-Butyl-N- (2-methylpropyl) amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino;
◆ C₁-C₆-Alkylthio: z.B. Methylthio, Ethylthio, Propylthio, 1-Methylethylthio, Butylthio, 1-Methylpropylthio, 2-Methylpropylthio und 1,1-Dimethylethylthio, Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
◆ C₁-C₆-Halogenalkylthio: einen C₁-C₆-Alkylthiorest wie voranstehend genannt, der partiell oder vollständig durch Fluor, Chlor, Brom und/oder Iod substituiert ist, also z.B. Fluormethylthio, Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2- chlorethylthio, 1-(Brommethyl)-2-bromethylthio, 4-Fluorbutylthio, 4-Chlorbutylthio, 4-Brombutylthio, Nonafluorbutylthio, 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio und Dodecafluorhexylthio;
◆ C₁-C₆-Alkylsulfinyl (C₁-C₆-Alkyl-S(=O)-): z.B. Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
◆ C₁-C₆-Halogenalkylsulfinyl: C₁-C₆-Alkylsulfinylrest wie voranstehend genannt, der partiell oder vollständig durch Fluor, Chlor, Brom und/oder Iod substituiert ist, also z.B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl, Nonafluorbutylsulfinyl, 5-Fluorpentylsulfinyl, 5-Chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl und Dodecafluorhexylsulfinyl;
◆ C₁-C₆-Alkylsulfonyl (C₁-C₆-Alkyl-S(=O)₂-): z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
◆ C₁-C₆-Halogenalkylsulfonyl: einen C₁-C₆-Alkylsulfonylrest wie voranstehend genannt, der partiell oder vollständig durch Fluor, Chlor, Brom und/oder Iod substituiert ist, also z.B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl und Dodecafluorhexylsulfonyl;

In einer besonderen Ausführungsform haben die Variablen der Verbindungen der Formel I folgende Bedeutungen, wobei diese für sich allein betrachtet als auch in der Kombination miteinander besondere Ausgestaltungen der Verbindungen der Formel I darstellen:

Bevorzugt sind die N-heterocyclylsubstituierten Thienyloxy-Pyrimidine der Formel I, in der die Variablen W, X, Y und Z CR³ bedeuten.

Bevorzugt sind die N-heterocyclylsubstituierten Thienyloxy-Pyrimidine der Formel I, in der ein oder zwei der Variablen W, X, Y, Z N bedeuten.

Ebenso bevorzugt sind die N-heterocyclylsubstituierten Thienyloxy-Pyrimidine der Formel I, in der eine der Variablen X oder Z oder zwei der Variablen W und Z oder X und Y N bedeuten.

Insbesondere bevorzugt sind die N-heterocyclylsubstituierten Thienyloxy-Pyrimidine der Formel I, in der zwei der Variablen W, X, Y, Z, besonders bevorzugt W und Z, N bedeuten.

Ebenso insbesondere bevorzugt sind die N-heterocyclylsubstituierten Thienyloxy-Pyrimidine der Formel I, in der die Variablen W und Z, oder W und X, oder W und Y N bedeuten.

Ebenso insbesondere bevorzugt sind die N-heterocyclylsubstituierten Thienyloxy-Pyrimidine der Formel I, in der eine der Variablen W, X, Y, Z, besonders bevorzugt W oder Z, N bedeuten.

Ebenso insbesondere bevorzugt sind die N-heterocyclylsubstituierten Thienyloxy-Pyrimidine der Formel I, in der die Variablen X oder Z N bedeuten.

Ganz besonders bevorzugt sind die N-heterocyclylsubstituierten Thienyloxy-Pyrimidine der Formel I, in der die Variablen W, Y und Z N oder CH und X CR³ bedeuten.

Ebenso insbesondere bevorzugt sind die N-heterocyclylsubstituierten Thienyloxy-Pyrimidine der Formel I, in der die Variablen Y oder Z N, und die Variablen W und X CR³ bedeuten.

Ebenso insbesondere bevorzugt sind die N-heterocyclylsubstituierten Thienyloxy-Pyrimidine der Formel I, in der die Variablen W CH, Y N oder CH, Z N oder CH und X CR³ bedeuten.

Ebenso bevorzugt sind die N-heterocyclylsubstituierten Thienyloxy-Pyrimidine der Formel I, in der die Variablen Y N und W,X,Z CR³ bedeuten.

Ebenso ganz besonders bevorzugt sind die N-heterocyclylsubstituierten Thienyloxy-Pyrimidine der Formel I, in der W N, X CR³, Y CH, Z CH bedeuten.

Ebenso ganz besonders bevorzugt sind die N-heterocyclylsubstituierten Thienyloxy-Pyrimidine der Formel I, in der W CH, X N, Y CR³, Z CR³ bedeuten.

Ebenso ganz besonders bevorzugt sind die N-heterocyclylsubstituierten Thienyloxy-Pyrimidine der Formel I, in der W CR³, X CR³, Y CH, Z N bedeuten.

Ebenso bevorzugt sind die N-heterocyclylsubstituierten Thienyloxy-Pyrimidine der Formel I, in der
- R¹: Wasserstoff, Halogen, Cyano, C₁-C₆-Alkyl, C₁-C₆-Halogenalkyl oder C₁-C₆-Alkoxy;
besonders bevorzugt Wasserstoff, Halogen wie Fluor, Chlor oder Brom, C₁-C₆-Alkyl, wie Methyl oder Ethyl; insbesondere bevorzugt Wasserstoff, Fluor, Chlor oder Methyl;
bedeutet.

Ebenso bevorzugt sind die N-heterocyclylsubstituierten Thienyloxy-Pyrimidine der Formel I, in der
- R¹: Wasserstoff, Halogen, Cyano, C₁-C₆-Halogenalkyl oder C₁-C₆-Alkoxy;
besonders bevorzugt Wasserstoff, Halogen wie Fluor, Chlor oder Brom;
insbesondere bevorzugt Wasserstoff, Fluor oder Chlor;
bedeutet.

Ebenso bevozrugt sind die N-heterocyclylsubstituierten Thienyloxy-Pyrimidine der Formel I, in der
- R¹: Wasserstoff oder C₁-C₆-Alkoxy, wie z.B. Methoxy oder Ethoxy;
besonders bevorzugt Wasserstoff oder Methoxy;
insbesondere bevorzugt Methoxy;
bedeutet.

Ebenso bevorzugt sind die N-heterocyclylsubstituierten Thienyloxy-Pyrimidine der Formel I, in der
- R²: Wasserstoff, Halogen, Cyano, C₁-C₆-Alkyl, C₁-C₆-Halogenalkyl, C₁-C₆-Alkoxy, C₁-C₆-Halogenalkoxy, C₁-C₆-Alkoxy-C₁-C₄-alkyl, C₁-C₆-Alkylthio oder COOR⁷; besonders bevorzugt Wasserstoff, Halogen, Cyano, C₁-C₆-Alkyl, C₁-C₆-Halogenalkyl, C₁-C₆-Alkoxy, C₁-C₆-Alkoxy-C₁-C₄-alkyl oder C₁-C₆-Alkylthio;
insbesondere bevorzugt Wasserstoff, Halogen, wie Fluor, Chlor oder Brom, C₁-C₆-Alkyl, wie Methyl oder Ethyl, oder C₁-C₆-Halogenalkyl, wie Trifluormethyl, Trichlormethyl oder Difluormethyl;
sehr bevorzugt Wasserstoff, Fluor, Chlor, Methyl oder Trifluormethyl;
bedeutet.

Ebenso bevorzugt sind die N-heterocyclylsubstituierten Thienyloxy-Pyrimidine der Formel I, in der
- R²: Wasserstoff, Halogen, wie Fluor, Chlor oder Brom, C₁-C₆-Alkyl, wie Methyl oder Ethyl, C₁-C₆-Halogenalkyl, wie Trifluormethyl, Trichlormethyl oder Difluormethyl, oder C₁-C₆-Alkoxy, wie Methoxy oder Ethoxy;
besonders bevorzugt Wasserstoff, Fluor, Chlor, Methyl, Trifluormethyl oder Methoxy;
bedeutet.

Ebenso bevorzugt sind die N-heterocyclylsubstituierten Thienyloxy-Pyrimidine der Formel I, in der
- R²: Halogen, Cyano, C₁-C₆-Halogenalkyl, C₁-C₆-Alkoxy, C₁-C₆-Halogenalkoxy, C₁-C₆-Alkoxy-C₁-C₆-alkyl, C₁-C₆-Alkylthio oder COOR⁷;
besonders bevorzugt Halogen, Cyano, C₁-C₆-Halogenalkyl, C₁-C₆-Alkoxy, C₁-C₆-Alkoxy-C₁-C₆-alkyl oder C₁-C₆-Alkylthio;
insbesondere bevorzugt Halogen wie Fluor, Chlor oder Brom, C₁-C₆-Halogenalkyl wie Trifluormethyl, Trichlormethyl oder Difluormethyl, oder C₁-C₆-Alkoxy wie Methoxy oder Ethoxy;
sehr bevorzugt Fluor, Chlor, Trifluormethyl oder Methoxy;
bedeutet.

Ebenso bevorzugt sind die N-heterocyclylsubstituierten Thienyloxy-Pyrimidine der Formel I, in der
- R³: Wasserstoff, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Alkylsulfonyl oder COOR⁷; besonders bevorzugt Wasserstoff, Halogen, Cyano, C₁-C₆-Alkyl, C₁-C₆-Halogenalkyl oder C₁-C₆-Alkoxy;
insbesondere bevorzugt Wasserstoff, Halogen, wie Fluor, Chlor oder Brom, oder C₁-C₆-Halogenalkyl, wie Trifluormethyl, Trichlormethyl oder Difluormethyl; sehr bevorzugt Wasserstoff, Fluor, Chlor oder Trifluormethyl;
bedeutet.

Ebenso bevorzugt sind die N-heterocyclylsubstituierten Thienyloxy-Pyrimidine der Formel I, in der
- R³: Wasserstoff, Halogen, Cyano, C₁-C₆-Alkyl, C₁-C₆-Alkoxy, C₁-C₆-Halogenalkoxy, C₁-C₆-Alkylthio, C₁-C₆-Alkylsulfonyl oder COOR⁷;
besonders bevorzugt Wasserstoff, Halogen, Cyano, C₁-C₆-Alkyl, C₁-C₆-Alkoxy oder C₁-C₆-Halogenalkoxy;
insbesondere bevorzugt Wasserstoff, Halogen, wie Fluor, Chlor oder Brom, oder C₁-C₄-Halogenalkoxy wie Difluormethoxy oder Trifluormethoxy;
sehr bevorzugt Wasserstoff, Fluor, Chlor, Difluormethoxy oder Trifluormethoxy;
bedeutet.

Ebenso bevorzugt sind die N-heterocyclylsubstituierten Thienyloxy-Pyrimidine der Formel I, in der jeweils unabhängig voneinander
- R⁴, R⁵, R⁶: Wasserstoff, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Alkylsulfonyl, C₁-C₆-Halogenalkylsufonyl;
besonders bevorzugt Wasserstoff, Halogen, Cyano, C₁-C₆-Alkyl, C₁-C₆-Halogenalkyl, C₁-C₆-Halogenalkoxy, C₁-C₆-Alkylsulfonyl oder C₁-C₆-Halogenalkylsufonyl;
insbesondere bevorzugt Wasserstoff, Halogen, wie Fluor, Chlor oder Brom, C₁-C₆-Halogenalkyl, wie Trifluormethyl, Trichlormethyl oder Difluormethyl, C₁-C₆-Alkylsulfonyl, wie Methylsulfonyl oder Ethylsulfonyl, oder C₁-C₆-Halogenalkylsufonyl, wie Trifluormethylsulfonyl, Trichlormethylsulfonyl oder Difluormethylsulfonyl ;
sehr bevorzugt Wasserstoff, Fluor, Chlor, Trifluormethyl oder Methylsulfonyl;
bedeuten.

Ebenso bevorzugt sind die N-heterocyclylsubstituierten Thienyloxy-Pyrimidine der Formel I, in der jeweils unabhängig voneinander
- R⁴, R⁵, R⁶: Wasserstoff, Halogen, Cyano, C₁-C₆-Alkyl, C₁-C₆-Alkoxy, C₁-C₆-Halogenalkoxy, C₁-C₆-Alkylthio, C₁-C₆-Alkylsulfonyl, C₁-C₆-Halogenalkylsufonyl; besonders bevorzugt Wasserstoff, Halogen, Cyano, C₁-C₆-Alkyl, C₁-C₆-Halogenalkoxy, C₁-C₆-Alkylsulfonyl oder C₁-C₆-Halogenalkylsufonyl;
insbesondere bevorzugt Wasserstoff, Halogen, wie Fluor, Chlor oder Brom, C₁-C₆-Halogenalkoxy wie Difluormethoxy, Trifluormethoxy, C₁-C₆-Alkylsulfonyl wie Methylsulfonyl oder Ethylsulfonyl, oder C₁-C₆-Halogenalkylsulfonyl, wie Trifluormethylsulfonyl, Trichlormethylsulfonyl oder Difluormethylsulfonyl;
sehr bevorzugt Wasserstoff, Fluor, Chlor, Difluormethoxy, Trifluormethoxy oder Methylsulfonyl;
bedeuten.

Ebenso bevorzugt sind die N-heterocyclylsubstituierten Thienyloxy-Pyrimidine der Formel I, in der R⁶ Wasserstoff und jeweils unabhängig voneinander
- R⁴, R⁵: Wasserstoff, Halogen, C₁-C₆-Alkyl oder C₁-C₆-Halogenalkyl;
besonders bevorzugt Wasserstoff, Chlor, Methyl oder Trifluormethyl;
bedeuten.

Insbesondere ebenso bevorzugt sind die N-heterocyclylsubstituierten Thienyloxy-Pyrimidine der Formel I, in der der Thienylrest in 3-Position über das Sauerstoffatom mit dem Pyrimidingerüst verknüpft und mit R⁴ und R⁵ in 4 bzw. 5-Position substituiert ist.

Ebenso insbesondere bevorzugt sind die N-heterocyclylsubstituierten Thienyloxy-Pyrimidine der Formel I, in der der Thienylrest in 2-Position über das Sauerstoffatom mit dem Pyrimidingerüst verknüpft und mit R⁴ und R⁵ in 4 bzw. 5-Position substituiert ist.

Ebenso bevorzugt sind die N-heterocyclylsubstituierten Thienyloxy-Pyrimidine der Formel I, in der R⁵ und R⁶ Wasserstoff und
- R⁴: Halogen, Cyano, C₁-C₆-Alkyl, C₁-C₆-Halogenalkyl oder C₁-C₆-Alkoxy;
besonders bevorzugt Halogen oder C₁-C₆-Halogenalkyl;
sehr bevorzugt Fluor, Chlor oder Trifluormethyl;
bedeutet.

Ebenso bevorzugt sind die N-heterocyclylsubstituierten Thienyl oxy-Pyrimidine der Formel I, in der R⁵ und R⁶ Wasserstoff und
- R⁴: Halogen, Cyano, C₁-C₆-Alkyl oder C₁-C₆-Alkoxy; besonders bevorzugt Halogen; sehr bevorzugt Fluor oder Chlor;
bedeutet.

Insbesondere ebenso bevorzugt sind die N-heterocyclylsubstituierten Thienyloxy-Pyrimidine der Formel I, in der der Thienylrest in 3-Position über das Sauerstoffatom mit dem Pyrimidingerüst verknüpft und mit R⁴ in 5-Position substituiert ist.

Insbesondere ebenso bevorzugt sind die N-heterocyclylsubstituierten Thienyloxy-Pyrimidine der Formel I, in der der Thienylrest in 3-Position über das Sauerstoffatom mit dem Pyrimidingerüst verknüpft ist, R⁵ und R⁶ Wasserstoff bedeuten und der Thienylrest in 5-Position mit R⁴ substituiert ist, wobei
- R⁴: Halogen, Cyano, C₁-C₆-Alkyl, Trichlormethyl, Difluormethyl, Monofluormethyl, C₁-C₆-Alkoxy oder C₁-C₆-Halogenalkoxy; besonders bevorzugt Halogen, Trichlormethyl, Difluormethyl, Monofluormethyl; Difluormethoxy oder Trifluormethoxy, sehr bevorzugt Fluor, Chlor, Trichlormethyl, Difluormethyl, Monofluormethyl, Difluormethoxy oder Trifluormethoxy;
bedeutet.

Insbesondere ebenso bevorzugt sind die N-heterocyclylsubstituierten Thienyloxy-Pyrimidine der Formel I, in der der Thienylrest in 2-Position über das Sauerstoffatom mit dem Pyrimidingerüst verknüpft und mit R⁴ in 5-Position substituiert ist.

Ebenso bevorzugt sind die N-heterocyclylsubstituierten Thienyloxy-Pyrimidine der Formel I, in der
- R¹: Wasserstöff oder C₁-C₆-Alkyl;
- R²: Wasserstoff, Halogen, Cyano, C₁-C₆-Alkyl, C₁-C₆-Halogenalkyl, C₁-C₆-Alkoxy, C₁-C₆-Alkylthio, C₁-C₆-Alkoxy-C₁-C₄-alkyl oder COOR⁷;
bedeutet.

Ebenso bevorzugt sind die N-heterocyclylsubstituierten Thienyl oxy-Pyrimidine der Formel I, in der
- R¹: Wasserstoff; und
- R²: Halogen, Cyano, C₁-C₆-Halogenalkyl, C₁-C₆-Alkoxy, C₁-C₆-Alkylthio, C₁-C₆-Alkoxy-C₁-C₄-alkyl oder COOR⁷:
bedeutet.

Insbesondere ebenso bevorzugt sind die N-heterocyclylsubstituierten Thienyloxy-Pyrimidine der Formel I, in der
- R¹: C₁-C₆-Alkoxy; besonders bevorzugt C₁-C₄-Alkoxy, wie z.B. Methoxy oder Ethoxy;
insbesondere bevorzugt Methoxy; und
- R²: Wasserstoff
bedeutet.

Insbesondere ebenso bevorzugt sind die N-heterocyclylsubstituierten Thienyloxy-Pyrimidine der Formel I, in der
- R¹: Wasserstoff oder C₁-C₆-Alkoxy; besonders bevorzugt Wasserstoff oder C₁-C₄-Alkoxy, wie z.B. Methoxy oder Ethoxy;
insbesondere bevorzugt Wasserstoff oder Methoxy; und
- R²: Wasserstoff
bedeutet.

Ebenso bevorzugt sind die N-heterocyclylsubstituierten Thienyl oxy-Pyrimidine der Formel I, in der der Thienylrest in 3-Position über das Sauerstoffatom mit dem Pyrimidingerüst verknüpft ist und die Variablen Y oder Z N, und W und X CR³ bedeuten.

Ebenso bevorzugt sind die N-heterocyclylsubstituierten Thienyl oxy-Pyrimidine der Formel I, in der Thienylrest in 3-Position über das Sauerstoffatom mit dem Pyrimidingerüst verknüpft ist und die Variabler W CR³, Y N oder CR³, Z N oder CR³, und X CR³ bedeuten.

Insbesondere ebenso bevorzugt sind die N-heterocyclylsubstituierten Thienyloxy-Pyrimidine der Formel I, in der Thienylrest in 3-Position über das Sauerstoffatom mit dem Pyrimidingerüst verknüpft ist und mit R⁴ in 5-Position substituiert ist, wobei
- R¹: Wasserstoff, C₁-C₆-Alkyl, wie z.B. Methyl, oder C₁-C₆-Alkoxy, wie z.B. Methoxy,
besonders bevorzugt Wasserstoff, Methyl oder Methoxy, insbesondere bevorzugt Methyl;
- R²: Wasserstoff;
- R⁴: C₁-C₆-Halogenalkyl, besonders bevorzugt Trifluormethyl oder Difluormethyl;
- R⁵, R⁶: Wasserstoff;
und die Variablen W CR³, X CR³, Y CH, und Z N bedeuten; wobei
- R³: Wasserstoff oder Halogen, bevorzugt Wasserstoff oder Chlor, insbesondere bevorzugt Chlor;
bedeutet.

Außerordentlich bevorzugt sind Verbindungen der Formel Ia (mit X = C-CF₃, R⁴ = 5-CF₃, R⁵ = H, R⁶ = H; der Thienylrest ist in 3-Position über ein Sauerstoffatom mit dem Pyrimidingerüst verknüpft), insbesondere die Verbindungen Ia.1 bis Ia.121 der Tabelle 1, wobei die Definitionen der Variablen W, Y, Z, R¹ und R² nicht nur in Kombination miteinander, sondern auch jeweils für sich allein betrachtet für die erfindungsgemäßen Verbindungen eine besondere Rolle spielen.

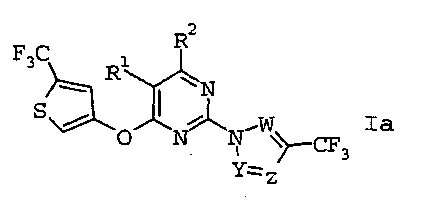

**Tabelle 1**

| Nr. | R¹ | R² | W | Y | Z |
|---|---|---|---|---|---|
| Ia.1 | H | H | CH | CH | CH |
| Ia.2 | H | H | N | CH | CH |
| Ia.3 | H | H | CH | N | CH |
| Ia.4 | H | H | CH | CH | N |
| Ia.5 | H | H | N | CH | N |
| Ia.6 | H | H | N | N | N |
| Ia.7 | CH₃ | CH₃ | CH | CH | CH |
| Ia.8 | CH₃ | CH₃ | N | CH | CH |
| Ia.9 | CH₃ | CH₃ | CH | N | CH |
| Ia.10 | CH₃ | CH₃ | CH | CH | N |
| Ia.11 | CH₃ | CH₃ | N | CH | N |
| Ia. 12 | CH₃ | CH₃ | N | N | N |
| Ia.13 | H | OCH₃ | CH | CH | CH |
| Ia.14 | H | OCH₃ | N | CH | CH |
| Ia.15 | H | OCH₃ | CH | N | CH |
| Ia.16 | H | OCH₃ | CH | CH | N |
| Ia.17 | H | OCH₃ | N | CH | N |
| Ia.18 | H | OCH₃ | N | N | N |
| Ia.19 | H | CN | CH | CH | CH |
| Ia.20 | H | CN | N | CH | CH |
| Ia.21 | H | CN | CH | N | CH |
| Ia.22 | H | CN | CH | CH | N |
| Ia.23 | H | CN | N | CH | N |
| Ia.24 | H | CN | N | N | N |
| Ia.25 | H | SCH₃ | CH | CH | CH |
| Ia.26 | H | SCH₃ | N | CH | CH |
| Ia.27 | H | SCH₃ | CH | N | CH |
| Ia.28 | H | SCH₃ | CH | CH | N |
| Ia.29 | H | SCH₃ | N | CH | N |
| Ia.30 | H | SCH₃ | N | N | N |
| Ia.31 | H | CF₃ | CH | CH | CH |
| Ia.32 | H | CF₃ | N | CH | CH |
| Ia.33 | H | CF₃ | CH | N | CH |
| Ia.34 | H | CF₃ | CH | CH | N |
| Ia.35 | H | CF₃ | N | CH | N |
| Ia.36 | H | CF₃ | N | N | N |
| Ia.37 | H | Cl | CH | CH | CH |
| Ia.38 | H | Cl | N | CH | CH |
| Ia.39 | H | Cl | CH | N | CH |
| Ia.40 | H | Cl | CH | CH | N |
| Ia.41 | H | Cl | N | CH | N |
| Ia.42 | H | Cl | N | N | N |
| Ia.43 | H | Br | CH | CH | CH |
| Ia.44 | H | Br | N | CH | CH |
| Ia.45 | H | Br | CH | N | CH |
| Ia.46 | H | Br | CH | CH | N |
| Ia.47 | H | Br | N | CH | N |
| Ia.48 | H | Br | N | N | N |
| Ia.49 | H | F | CH | CH | CH |
| Ia.50 | H | F | N | CH | CH |
| Ia.51 | H | F | CH | N | CH |
| Ia.52 | H | F | CH | CH | N |
| Ia.53 | H | F | N | CH | N |
| Ia.54 | H | F | N | N | N |
| Ia.55 | H | CH₂OCH₃ | CH | CH | CH |
| Ia.56 | H | CH₂OCH₃ | N | CH | CH |
| Ia.57 | H | CH₂OCH₃ | CH | N | CH |
| Ia.58 | H | CH₂OCH₃ | CH | CH | N |
| Ia.59 | H | CH₂OCH₃ | N | CH | N |
| Ia.60 | H | CH₂OCH₃ | N | N | N |
| Ia.61 | H | CO₂C₂H₅ | CH | CH | CH |
| Ia.62 | H | CO₂C₂H₅ | N | CH | CH |
| Ia.63 | H | CO₂C₂H₅ | CH | N | CH |
| Ia.64 | H | CO₂C₂H₅ | CH | CH | N |
| Ia.65 | H | CO₂C₂H₅ | N | CH | N |
| Ia.66 | H | CO₂C₂H₅ | N | N | N |
| Ia.67 | CH₃ | H | CH | CH | CH |
| Ia.68 | CH₃ | H | N | CH | CH |
| Ia.69 | CH₃ | H | CH | N | CH |
| Ia.70 | CH₃ | H | CH | CH | N |
| Ia.71 | CH₃ | H | N | CH | N |
| Ia.72 | CH₃ | H | N | N | N |
| Ia.73 | H | CH₃ | CH | CH | CH |
| Ia.74 | H | CH₃ | N | CH | CH |
| Ia.75 | H | CH₃ | CH | N | CH |
| Ia.76 | H | CH₃ | CH | CH | N |
| Ia.77 | H | CH₃ | N | CH | N |
| Ia.78 | H | CH₃ | N | N | N |
| Ia.79 | C₂H₅ | H | CH | CH | CH |
| Ia.80 | C₂H₅ | H | N | CH | CH |
| Ia.81 | C₂H₅ | H | CH | N | CH |
| Ia.82 | C₂H₅ | H | CH | CH | N |
| Ia.83 | C₂H₅ | H | N | CH | N |
| Ia.84 | C₂H₅ | H | N | N | N |
| Ia.85 | CF₃ | H | CH | CH | CH |
| Ia.86 | CF₃ | H | N | CH | CH |
| Ia.87 | CF₃ | H | CH | N | CH |
| Ia.88 | CF₃ | H | CH | CH | N |
| Ia.89 | CF₃ | H | N | CH | N |
| Ia.90 | CF₃ | H | N | N | N |
| Ia.91 | Cl | H | CH | CH | CH |
| Ia.92 | Cl | H | N | CH | CH |
| Ia.93 | Cl | H | CH | N | CH |
| Ia.94 | Cl | H | C_{H} | C_{H} | N |
| Ia.95 | Cl | H | N | CH | N |
| Ia.96 | Cl | H | N | N | N |
| Ia.97 | H₃CO | H | CH | CH | CH |
| Ia.98 | H₃CO | H | N | CH | CH |
| Ia.99 | H₃CO | H | CH | N | CH |
| Ia.100 | H₃CO | H | CH | CH | N |
| Ia.101 | H₃CO | H | N | CH | N |
| Ia.102 | H₃CO | H | N | N | N |
| Ia.103 | H | H | N | N | CH |
| Ia.104 | CH₃ | CH₃ | N | N | CH |
| Ia.105 | H | OCH₃ | N | N | CH |
| Ia.106 | H | CN | N | N | CH |
| Ia.107 | H | SCH₃ | N | N | CH |
| Ia.108 | H | CF₃ | N | N | CH |
| Ia.109 | H | Cl | N | N | CH |
| Ia.110 | H | Br | N | N | CH |
| Ia.111 | H | F | N | N | CH |
| Ia.112 | H | CH₂OCH₃ | N | N | CH |
| Ia.113 | H | CO₂C₂H₅ | N | N | CH |
| Ia.114 | CH₃ | H | N | N | CH |
| Ia.115 | H | CH₃ | N | N | CH |
| Ia.116 | C₂H₅ | H | N | N | CH |
| Ia.117 | CF₃ | H | N | N | CH |
| Ia.118 | Cl | H | N | N | CH |
| Ia.119 | H₃CO | H | N | N | CH |
| Ia.120 | H | H | CH | N | CCH₃ |
| Ia.121 | H | H | CCl | CH | N |

Ebenso außerordentlich bevorzugt sind die Verbindungen der Formel Ib, insbesondere die Verbindungen Ib.1 bis Ib.121, die sich von den entsprechenden Verbindungen Ia.1 bis Ia.121 dadurch unterscheiden, daß R⁴ für Chlor steht.

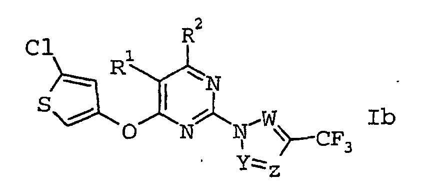

Ebenso außerordentlich bevorzugt sind die Verbindungen der Formel Ic, insbesondere die Verbindungen Ic.1 bis Ic.121, die sich von den entsprechenden Verbindungen Ia.1 bis Ia.121 dadurch unterscheiden, daß der Thienylrest in 2-Position über das Sauerstoffatom mit dem Pyrimidingerüst verknüpft ist.

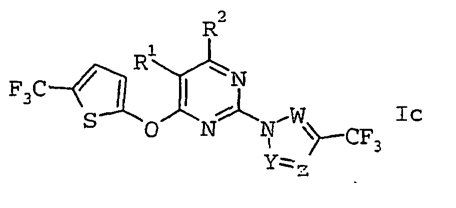

Ebenso außerordentlich bevorzugt sind die Verbindungen der Formel Id, insbesondere die Verbindungen Id.1 bis Id.121, die sich von den entsprechenden Verbindungen Ia.1 bis Ia.121 dadurch unterscheiden, daß R⁴ Chlor ist und der Thienylrest in 2-Position über das Sauerstoffatom mit dem Pyrimidingerüst verknüpft ist.

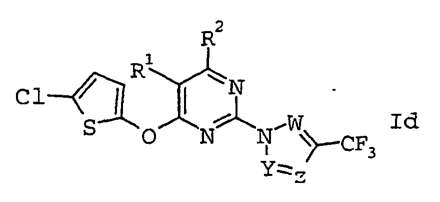

Ebenso außerordentlich bevorzugt sind die Verbindungen der Formel Ie, insbesondere die Verbindungen Ie.1 bis Ie.121, die sich von den entsprechenden.Verbindungen Ia.1 bis Ia.121 dadurch unterscheiden, daß R⁴ in 5-Position und R⁵ in 4-Position Chlor sind.

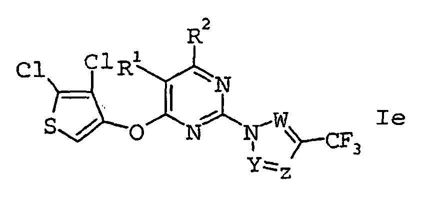

Ebenso außerordentlich bevorzugt sind die Verbindungen der Formel If, insbesondere die Verbindungen If.1 bis If.121, die sich von den entsprechenden Verbindungen Ia.1 bis Ia.121 dadurch unterscheiden, daß R⁴ in 5-Position und R⁵ in 2-Position Chlor sind.

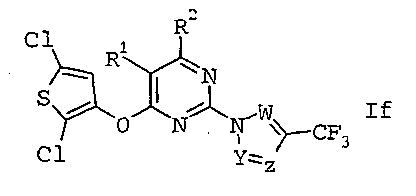

Ebenso außerordentlich bevorzugt sind die Verbindungen der Formel Ig, insbesondere die Verbindungen Ig.1 bis Ig.121, die sich von den entsprechenden Verbindungen Ia.1 bis Ia.121 dadurch unterscheiden, daß R⁴ in 5-Position und R⁵ in 4-Position Chlor sind und der Thienylrest in 2-Position über das Sauerstoffatom mit dem Pyrimidingerüst verknüpft ist

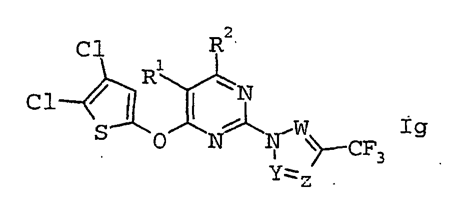

Ebenso außerordentlich bevorzugt sind die Verbindungen der Formel Ih, insbesondere die Verbindungen Ih.1 bis Ih.121, die sich von den entsprechenden Verbindungen Ia.1 bis Ia.121 dadurch unterscheiden, daß X C-NO₂ ist.

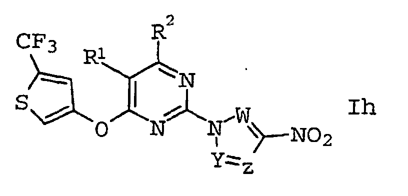

Ebenso außerordentlich bevorzugt sind die Verbindungen der Formel Ii, insbesondere die Verbindungen Ii.1 bis Ii.121, die sich von den entsprechenden Verbindungen Ia.1 bis Ia.121 dadurch unterscheiden, daß X C-Cl ist.

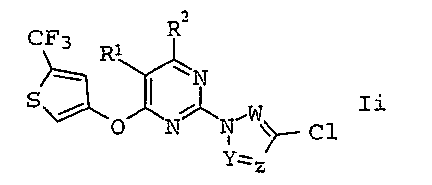

Ebenso außerordentlich bevorzugt sind die Verbindungen der Formel Ik, insbesondere die Verbindungen Ik.1 bis Ik.121, die sich von den entsprechenden Verbindungen Ia.1 bis Ia.121 dadurch unterscheiden, daß X NO₂ ist und für R⁴ für Chlor steht.

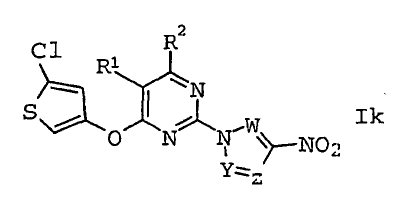

Ebenso außerordentlich bevorzugt sind die Verbindungen der Formel Il, insbesondere die Verbindungen Il.1 bis Il.121, die sich von den entsprechenden Verbindungen Ia.1 bis Ia.121 dadurch unterscheiden, daß X C-Cl ist und für R⁴ für Chlor steht.

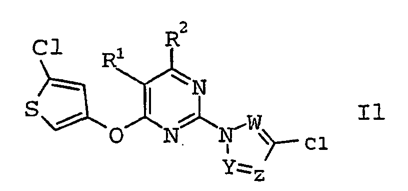

Ebenso außerordentlich bevorzugt sind die Verbindungen der Formel Im, insbesondere die Verbindungen Im.1 bis Im.121, die sich von den entsprechenden Verbindungen Ia.1 bis Ia.121 dadurch unterscheiden, daß X C-NO₂ ist und der Thienylrest in 2-Position über das Sauerstoffatom mit dem Pyrimidingerüst verknüpft ist.

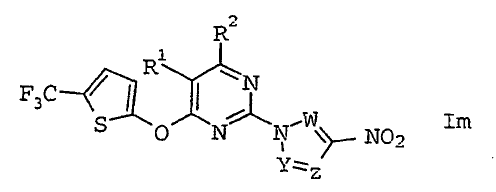

Ebenso außerordentlich bevorzugt sind die Verbindungen der Formel In, insbesondere die Verbindungen In.1 bis In.121, die sich von den entsprechenden Verbindungen Ia.1 bis Ia.121 dadurch unterscheiden, daß X C-Cl ist und der Thienylrest in 2-Position über das Sauerstoffatom mit dem Pyrimidingerüst verknüpft ist.

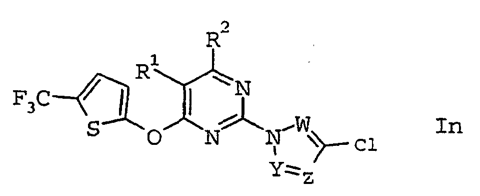

Ebenso außerordentlich bevorzugt sind die Verbindungen der Formel Io, insbesondere die Verbindungen Io.1 bis Io.121, die sich von den entsprechenden Verbindungen Ia.1 bis Ia.121 dadurch unterscheiden, daß X C-NO₂ ist, R⁴ für Chlor steht und der Thienylrest in 2-Position über das Sauerstoffatom mit dem Pyrimidingerüst verknüpft ist.

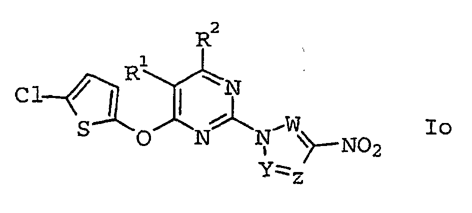

Ebenso außerordentlich bevorzugt sind die Verbindungen der Formel Ip, insbesondere die Verbindungen Ip.1 bis Ip.121, die sich von den entsprechenden Verbindungen Ia.1 bis Ia.121 dadurch unterscheiden, daß X C-Cl ist, R⁴ für Chlor steht und der Thienylrest in 2-Position über das Sauerstoffatom mit dem Pyrimidingerüst verknüpft ist.

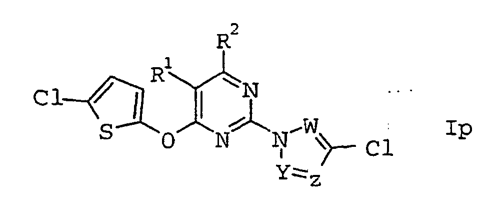

Außerdem bevorzugt sind die Verbindungen der Formel Iq (mit W C-CR³, R⁴ 5-CF₃, R⁵ H, R⁶ H; der Thienylrest ist in 3-Position über ein Sauerstoffatom mit dem Pyrimidingerüst verknüpft), insbesondere die Verbindungen Iq.1 bis Iq.205 der Tabelle 2, wobei die Definitionen der Variablen X, Y, Z, R¹, R² und R³ nicht nur in Kombination miteinander, sondern auch jeweils für sich allein betrachtet für die erfindungsgemäßen Verbindungen eine besondere Rolle spielen.

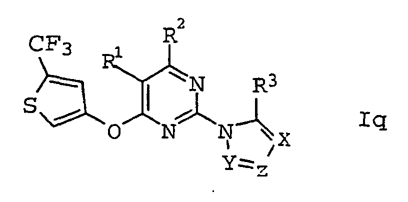

**Tabelle 2**

| Nr. | R¹ | R² | R³ | X | Y | Z |
|---|---|---|---|---|---|---|
| Iq.1 | H | H | CF₃ | CH | CH | CH |
| Iq. 2 | H | H | CF₃ | CH | N | CH |
| Iq.3 | H | H | CF₃ | CH | CH | N |
| Iq. 4 | H | H | CF₃ | N | CH | CH |
| Iq. 5 | H | H | CF₃ | N | CH | N |
| Iq. 6 | H | H | CF₃ | CH | N | N |
| Iq. 7 | H | H | CF₃ | N | N | CH |
| Iq. 8 | H | H | CF₃ | N | N | N |
| Iq.9 | H | H | CN | CH | CH | CH |
| Iq.10 | H | H | CN | CH | N | CH |
| Iq.11 | H | H | CN | CH | CH | N |
| Iq.12 | H | H | CN | N | CH | CH |
| Iq.13 | H | H | CN | N | CH | N |
| Iq.14 | H | H | CN | CH | N | N |
| Iq.15 | H | H | CN | N | N | CH |
| Iq.16 | H | H | CN | N | N | N |
| Iq.17 | H | H | C(=O)CF₃ | CH | CH | CH |
| Iq.18 | H | H | C(=O)CF₃ | CH | N | CH |
| Iq.19 | H | H | C(=O)CF₃ | CH | CH | N |
| Iq.20 | H | H | C(=O)CF₃ | N | CH | CH |
| Iq.21 | H | H | C(=O)CF₃ | N | CH | N |
| Iq.22 | H | H | C(=O)CF₃ | CH | N | N |
| Iq.23 | H | H | C(=O)CF₃ | N | N | CH |
| Iq.24 | H | H | C(=O)CF₃ | N | N | N |
| Iq.25 | H | H | CH₃ | CH | CCH₃ | CH |
| Iq.26 | H | H | CH₃ | N | CCH₃ | N |
| Iq.27 | H | H | CH₃ | N | CCH₃ | CH |
| Iq.28 | H | H | Cl | CH | CCl | CH |
| Iq.29 | H | H | Cl | N | CCl | N |
| Iq.30 | H | H | Cl | N | CCl | CH |
| Iq.31 | H | H | Br | C-tC₄H₉ | N | N |
| Iq.32 | H | H | Br | C-tC₄H₉ | CH | CH |
| Iq.33 | H | H | Br | C-tC₄H₉ | CH | N |
| Iq.34 | CH₃ | CH₃ | CF₃ | CH | CH | CH |
| Iq.35 | CH₃ | CH₃ | CF₃ | CH | N | CH |
| Iq.36 | CH₃ | CH₃ | CF₃ | CH | CH | N |
| Iq.37 | CH₃ | CH₃ | CF₃ | N | CH | CH |
| Iq.38 | CH₃ | CH₃ | CF₃ | N | CH | N |
| Iq.39 | CH₃ | CH₃ | CF₃ | CH | N | N |
| Iq.40 | CH₃ | CH₃ | CF₃ | N | N | CH |
| Iq.41 | CH₃ | CH₃ | CF₃ | N | N | N |
| Iq.42 | CH₃ | CH₃ | CN | CH | CH | CH |
| Iq.43 | CH₃ | CH₃ | CN | CH | N | CH |
| Iq.44 | CH₃ | CH₃ | CN | CH | CH | N |
| Iq.45 | CH₃ | CH₃ | CN | N | CH | CH |
| Iq.46 | CH₃ | CH₃ | CN | N | CH | N |
| Iq.47 | CH₃ | CH₃ | CN | CH | N | N |
| Iq.48 | CH₃ | CH₃ | CN | N | N | CH |
| Iq.49 | CH₃ | CH₃ | CN | N | N | N |
| Iq.50 | CH₃ | CH₃ | C(=O)CF₃ | CH | CH | CH |
| Iq.51 | CH₃ | CH₃ | C(=O)CF₃ | CH | N | CH |
| Iq.52 | CH₃ | CH₃ | C(=O)CF₃ | CH | CH | N |
| Iq.53 | CH₃ | CH₃ | C(=O)CF₃ | N | CH | CH |
| Iq.54 | CH₃ | CH₃ | C(=O)CF₃ | N | CH | N |
| Iq.55 | CH₃ | CH₃ | C(=O)CF₃ | CH | N | N |
| Iq.56 | CH₃ | CH₃ | C(=O)CF₃ | N | N | CH |
| Iq.57 | CH₃ | CH₃ | C(=O)CF₃ | N | N | N |
| Iq.58 | CH₃ | CH₃ | CH₃ | CH | CCH₃ | CH |
| Iq.59 | CH₃ | CH₃ | CH₃ | N | CCH₃ | N |
| Iq.60 | CH₃ | CH₃ | CH₃ | N | CCH₃ | CH |
| Iq.61 | CH₃ | CH₃ | Cl | CH | CCl | CH |
| Iq.62 | CH₃ | CH₃ | Cl | N | CCl | N |
| Iq.63 | CH₃ | CH₃ | Cl | N | CCl | CH |
| Iq.64 | CH₃ | CH₃ | Br | C-tC₄H₉ | N | N |
| Iq.65 | CH₃ | CH₃ | Br | C-tC₄H₉ | CH | CH |
| Iq.66 | CH₃ | CH₃ | Br | C-tC₄H₉ | CH | N |
| Iq.67 | CH₃ | H | CF₃ | CH | CH | CH |
| Iq.68 | CH₃ | H | CF₃ | CH | N | CH |
| Iq.69 | CH₃ | H | CF₃ | CH | CH | N |
| Iq.70 | CH₃ | H | CF₃ | N | CH | CH |
| Iq.71 | CH₃ | H | CF₃ | N | CH | N |
| Iq.72 | CH₃ | H | CF₃ | CH | N | N |
| Iq.73 | CH₃ | H | CF₃ | N | N | CH |
| Iq.74 | CH₃ | H | CF₃ | N | N | N |
| Iq.75 | CH₃ | H | CN | CH | CH | CH |
| Iq.76 | CH₃ | H | CN | CH | N | CH |
| Iq.77 | CH₃ | H | CN | CH | CH | N |
| Iq.78 | CH₃ | H | CN | N | CH | CH |
| Iq.79 | CH₃ | H | CN | N | CH | N |
| Iq.80 | CH₃ | H | CN | CH | N | N |
| Iq.81 | CH₃ | H | CN | N | N | CH |
| Iq.82 | CH₃ | H | CN | N | N | N |
| Iq.83 | CH₃ | H | C(=O)CF₃ | CH | CH | CH |
| Iq.84 | CH₃ | H | C(=O)CF₃ | CH | N | CH |
| Iq.85 | CH₃ | H | C(=O)CF₃ | CH | CH | N |
| Iq.86 | CH₃ | H | C(=O)CF₃ | N | CH | CH |
| Iq.87 | CH₃ | H | C(=O)CF₃ | N | CH | N |
| Iq.88 | CH₃ | H | C(=O)CF₃ | CH | N | N |
| Iq.89 | CH₃ | H | C(=O)CF₃ | N | N | CH |
| Iq.90 | CH₃ | H | C(=O)CF₃ | N | N | N |
| Iq.91 | CH₃ | H | CH₃ | CH | CCH₃ | CH |
| Iq.92 | CH₃ | H | CH₃ | N | CCH₃ | N |
| Iq.93 | CH₃ | H | CH₃ | N | CCH₃ | CH |
| Iq.94 | CH₃ | H | Cl | CH | CCl | CH |
| Iq.95 | CH₃ | H | Cl | N | CCl | N |
| Iq.96 | CH₃ | H | Cl | N | CCl | CH |
| Iq.97 | CH₃ | H | Br | C-tC₄H₉ | N | N |
| Iq.98 | CH₃ | H | Br | C-tC₄H₉ | CH | CH |
| Iq.99 | CH₃ | H | Br | C-tC₄H₉ | CH | N |
| Iq.100 | H | CH₃ | CF₃ | CH | CH | CH |
| Iq.101 | H | CH₃ | CF₃ | CH | N | CH |
| Iq.102 | H | CH₃ | CF₃ | CH | CH | N |
| Iq.103 | H | CH₃ | CF₃ | N | CH | CH |
| Iq.104 | H | CH₃ | CF₃ | N | CH | N |
| Iq.105 | H | CH₃ | CF₃ | CH | N | N |
| Iq.106 | H | CH₃ | CF₃ | N | N | CH |
| Iq.107 | H | CH₃ | CF₃ | N | N | N |
| Iq.108 | H | CH₃ | CN | CH | CH | CH |
| Iq.109 | H | CH₃ | CN | CH | N | CH |
| Iq.110 | H | CH₃ | CN | CH | CH | N |
| Iq.111 | H | CH₃ | CN | N | CH | CH |
| Iq.112 | H | CH₃ | CN | N | CH | N |
| Iq.113 | H | CH₃ | CN | CH | N | N |
| Iq.114 | H | CH₃ | CN | N | N | CH |
| Iq.115 | H | CH₃ | CN | N | N | N |
| Iq.116 | H | CH₃ | C(=O)CF₃ | CH | CH | CH |
| Iq.117 | H | CH₃ | C(=O)CF₃ | CH | N | CH |
| Iq.118 | H | CH₃ | C(=O)CF₃ | CH | CH | N |
| Iq.119 | H | CH₃ | C(=O)CF₃ | N | CH | CH |
| Iq.120 | H | CH₃ | C(=O)CF₃ | N | CH | N |
| Iq.121 | H | CH₃ | C(=O)CF₃ | CH | N | N |
| Iq.122 | H | CH₃ | C(=O)CF₃ | N | N | CH |
| Iq.123 | H | CH₃ | C(=O)CF₃ | N | N | N |
| Iq.124 | H | CH₃ | CH₃ | CH | CCH₃ | CH |
| Iq.125 | H | CH₃ | CH₃ | N | CCH₃ | N |
| Iq.126 | H | CH₃ | CH₃ | N | CCH₃ | CH |
| Iq.127 | H | CH₃ | Cl | CH | CCl | CH |
| Iq.128 | H | CH₃ | Cl | N | CCl | N |
| Iq.129 | H | CH₃ | Cl | N | CCl | CH |
| Iq.130 | H | CH₃ | Br | C-tC₄H₉ | N | N |
| Iq.131 | H | CH₃ | Br | C-tC₄H₉ | CH | CH |
| Iq.132 | H | CH₃ | Br | C-tC₄H₉ | CH | N |
| Iq.133 | OCH₃ | H | CF₃ | CH | CH | CH |
| Iq.134 | OCH₃ | H | CF₃ | CH | N | CH |
| Iq.135 | OCH₃ | H | CF₃ | CH | CH | N |
| Iq.136 | OCH₃ | H | CF₃ | N | CH | CH |
| Iq.137 | OCH₃ | H | CF₃ | N | CH | N |
| Iq.138 | OCH₃ | H | CF₃ | CH | N | N |
| Iq.139 | OCH₃ | H | CF₃ | N | N | CH |
| Iq.140 | OCH₃ | H | CF₃ | N | N | N |
| Iq.141 | OCH₃ | H | CN | CH | CH | CH |
| Iq.142 | OCH₃ | H | CN | CH | N | CH |
| Iq.143 | OCH₃ | H | CN | CH | CH | N |
| Iq.144 | OCH₃ | H | CN | N | CH | CH |
| Iq.145 | OCH₃ | H | CN | N | CH | N |
| Iq.146 | OCH₃ | H | CN | CH | N | N |
| Iq.147 | OCH₃ | H | CN | N | N | CH |
| Iq.148 | OCH₃ | H | CN | N | N | N |
| Iq.149 | OCH₃ | H | C(=O)CF₃ | CH | CH | CH |
| Iq.150 | OCH₃ | H | C(=O)CF₃ | CH | N | CH |
| Iq.151 | OCH₃ | H | C(=O)CF₃ | CH | CH | N |
| Iq.152 | OCH₃ | H | C(=O)CF₃ | N | CH | CH |
| Iq.153 | OCH₃ | H | C(=O)CF₃ | N | CH | N |
| Iq.154 | OCH₃ | H | C(=O)CF₃ | CH | N | N |
| Iq.155 | OCH₃ | H | C(=O)CF₃ | N | N | CH |
| Iq.156 | OCH₃ | H | C(=O)CF₃ | N | N | N |
| Iq.157 | OCH₃ | H | CH₃ | CH | CCH₃ | CH |
| Iq.158 | OCH₃ | H | CH₃ | N | CCH₃ | N |
| Iq.159 | OCH₃ | H | CH₃ | N | CCH₃ | CH |
| Iq.160 | OCH₃ | H | Cl | CH | CCl | CH |
| Iq.161 | OCH₃ | H | Cl | N | CCl | N |
| Iq.162 | OCH₃ | H | Cl | N | CCl | CH |
| Iq.163 | OCH₃ | H | Br | C-tC₄H₉ | N | N |
| Iq.164 | OCH₃ | H | Br | C-tC₄H₉ | CH | CH |
| Iq.165 | OCH₃ | H | Br | C-tC₄H₉ | CH | N |

Ebenso außerordentlich bevorzugt sind die Verbindungen der Formel Ir, insbesondere die Verbindungen Ir.1 bis Ir.165, die sich von den entsprechenden Verbindungen Iq.l bis Iq.165 dadurch unterscheiden, daß R⁴ für Chlor steht.

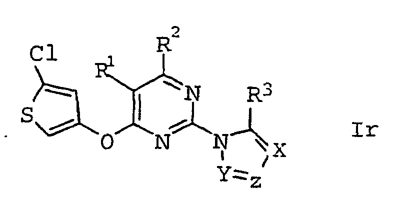

Ebenso außerordentlich bevorzugt sind die Verbindungen der Formel Is, insbesondere die Verbindungen Is.1 bis Is.165, die sich von den entsprechenden Verbindungen Iq.1 bis Iq.165 dadurch unterscheiden, daß der Thienylrest in 2-Position über das Sauerstoffatom mit dem Pyrimidingerüst verknüpft ist.

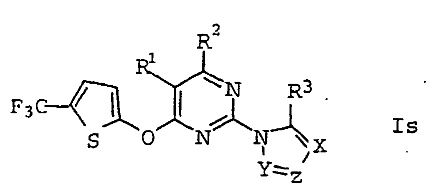

Ebenso außerordentlich bevorzugt sind die Verbindungen der Formel I, insbesondere die Verbindungen It.1 bis It.165, die sich von den entsprechenden Verbindungen Iq.1 bis Iq.165 dadurch unterscheiden, daß R⁴ Chlor ist und der Thienylrest in 2-Position über das Sauerstoffatom mit dem Pyrimidingerüst verknüpft ist.

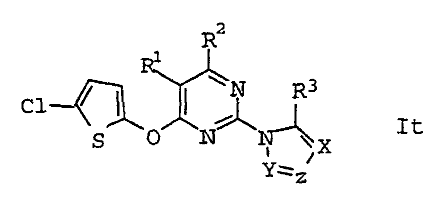

Die N-heterocyclylsubstituierten Thienyloxy-Pyrimidine der Formel I sind auf verschiedene Art und Weise erhältlich, beispielsweise nach folgenden Verfahren.

### Verfahren A

Dihaloheterocyclen der Formel VIII erhält man beispielsweise ausgehend von Dicarbonylheterocyclen, welche mit einem Chlorierungsmittel umgesetzt werden. Dichloroheterocyclen der Formel VIII können auch käuflich erworben werden.

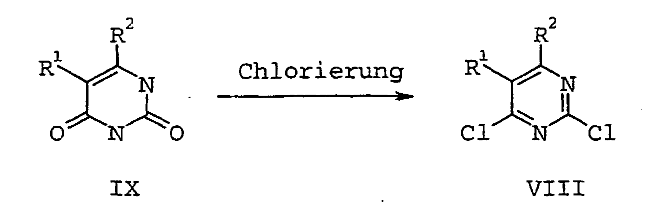

Diese Umsetzung erfolgt üblicherweise bei Temperaturen von 25 °C bis 130 °C in Gegenwart einer Base [vgl. Advances in Heterocyclic Chemistry, Hrsg. A. R. Katritzky, 1993, 58, 301-305; Heterocyclic Compounds, Hrsg. R. C. Ederfield, 1057, 6, 265-270].

Geeignete Chlorierungsmittel sind beispielsweise Phosphoroxychlorid, in Substanz oder in Anwesendheit eines Lösungsmittels oder Sulforylchlorid.

Als Basen kommen allgemein organische Basen, z.B. tertiäre Amine wie Trimethylamin, Triethylamin, Diisopropylethylamin, N,N-Dimethylanilin und N-Methylpiperidin, Pyridin, substituierte Pyridine wie Collidin, Lutidin und 4-Dimethylaminopyridin sowie bicyclische Amine in Betracht. Besonders bevorzugt wird N,N-Dimethylanilin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann vorteilhaft sein, das Chlorierungsmittel in einem Überschuß bezogen auf IX einzusetzen.

Die für die Herstellung der Verbindungen I benötigten Ausgangsstoffe sind in der Literatur bekannt, können in Analogie zu literaturbekannten Methoden hergestellt werden [E. Larsen et al.,

Synthesis 1995, 8, 934-936; JP-56139467; Organic Synthesis 1943, II, 422] oder sind käuflich erwerbbar.

Anschließend werden die Dihaloheterocyclen der Formel VIII mit Natrium- oder Kaliummethylmercaptan zu Pyrimidinen der Formel VII umgesetzt.

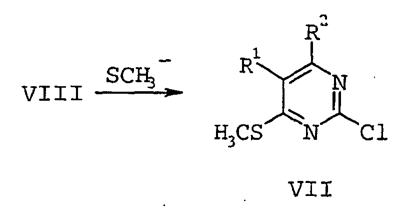

Diese Umsetzung erfolgt üblicherweise bei Temperaturen von 0 °C bis 80 °C in einem inerten organischen Lösungsmittel [vgl. WO 98/40379].

Geeignete Lösungsmittel sind Ether wie Diethylether, Diisopropylether, tert.-Butylmethylether, Dioxan, Anisol und Tetrahydrofuran. Bevorzugt ist Tetrahydrofuran.
Es können auch Gemische der genannten Lösungsmittel verwendet werden.

Die Edukte werden im allgemeinen in äquimolaren Mengen miteinander umgesetzt.

Das Mercaptan kann auch in situ durch die Umsetzung des entsprechenden Alkoholats mit Methylthiol in Methanol bei Raumtemperatur erzeugt werden [Recl. Trav. Chim. Pays-Bas 1942, 61, 291]. Pyrimidine der Formel VII können auch auf folgendem Weg hergestellt werden: 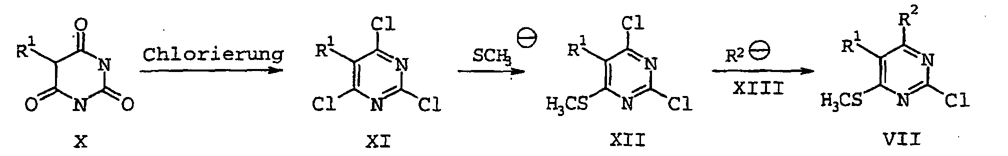 Käuflich zu erwerbende Tricarbonylverbindungen der Formel X werden mit Chlorierungsreagenzien, wie z.B. Phosphoroxychlorid, Sulforylchlorid oder Benzolphosphonsäuredichlorid in Gegenwart einer organischen Base, wie z.B. Triethylamin oder N,N-Dimethylaminopyridin, zum entsprechenden Trichloropyrimidin umgesetzt [vgl. DE 196 51 310, M. M. Robinson, J. Am. Chem. Soc. 1058, 80, 5481].

Anschließend setzt man die Trichloroverbindung der Formel XI mit einem Äquivalent Natrium- oder Kaliummercaptan in einem inerten organischen Lösungsmittel, wie z.B. Tetrahydrofuran oder Dioxan bei Temperaturen zwischen 0 °C und 80 °C zum entsprechenden Thioether der Formel XII um. Dabei kann das Mercaptan auch wie oben beschrieben in situ erzeugt werden.

Der Thioether der Formel XII wird dann mit einem Äquivalent des entsprechenden Nucleophils R^{2⊖} der Formel XIII umgesetzt. Für R²= Alkoxy, Alkenyloxy, Alkinyloxy, Halogenalkoxy, Alkoxyalkyl, Alkylamino, Dialkylamino, Alkylthio, Halogenalkylthio werden die entsprechenden Alkohole, Amine, Thiole gegebenenfalls unter dem Einfluß einer Base, wie z.B. einem Alkali- oder Erdalkalimetallcarbonat oder einem entsprechenden Hydroxid, in einem inerten organischen Lösungsmittel, wie z.B. Tetrahydrofuran, Acetonitril oder N,N-Dimethylformamid bei Temperaturen zwischen 0 °C und 130 °C zu Pyrimidinen der Formel VII umgesetzt [J. March, Advanced organic chemistry 1992, 641 f.].

Im Falle von R²=Halogen oder Cyano werden die entsprechenden Metallsalze R²-M eingesetzt.

Im Falle von R²= Alkyl werden die entsprechenden metallorganischen Verbindungen wie Grignard oder Organo-Lithium-Verbindungen eingesetzt.

Die auf diesen Wegen erhaltenen Pyrimidine der Formel VII setzt man mit Azolen der Formel V zu N-heterocyclylsubstituierten Pyrimidinen der Formel VI um:

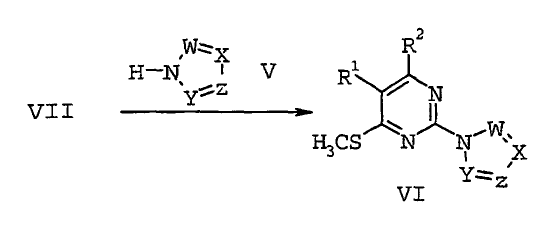

Diese Umsetzung erfolgt üblicherweise bei Temperaturen von 0 °C bis 130 °C in einem inerten organischen Lösungsmittel in Gegenwart einer Base [vgl. WO 98/40379].

Geeignete Lösungsmittel sind beispielsweise Ether wie Diethylether, Diisopropylether, tert.-Butylmethylether, Dioxan, Anisol und Tetrahydrofuran, Nitrile wie Acetonitril und Propionitril,

Dimethylformamid oder Dimethylsulfoxid. Bevorzugt ist Dimethylformamid.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat in Betracht. Bevorzugt sind Alkalimetall- und Erdalkalimetallcarbonate, z.B. Kaliumcarbonat.

Die Basen werden im allgemeinen in äquimolaren Mengen oder im Überschuß eingesetzt.

Des weiteren kann es von Vorteil sein, die Umsetzung in Gegenwart katalytischer Menge, z.B. 0.1 Äq., einer Base wie z.B. DABCO (1,4-Diazabicyclo[2.22]octan) durchzuführen (vgl. J.A. Lin, E.W. McLean, J.L. Kelley, J.Chem.Soc., Chem. Comm. 1994, 8, 913-914.

Die Edukte werden im allgemeinen in äquimolaren Mengen miteinander umgesetzt.

Dann werden die N-heterocyclylsubstituierten Pyrimidine der Formel VI mit einem Oxidationsmittel zu Verbindungen der Formel II umgesetzt:

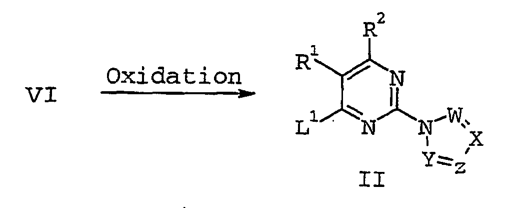

Diese Umsetzung erfolgt üblicherweise bei Temperaturen von 0 °C bis 60 °C in einem inerten organischen Lösungsmittel [vgl. J March, Advanced Organic Chemistry, 1992, 1201-1203.].

L¹ steht für eine nucleophil verdrängbare Abgangsgruppe, z.B. Alkylsulfonyl, bevorzugt Methylsulfonyl.

Geeignete Oxidationsmittel sind Metachlorperbenzoesäure, Wasserstoffperoxid, Natriumperiodat oder Oxon®. Bevorzugt ist Meta chlorperbenzoesäure.

Es kann von Vorteil sein, die Umsetzung in Gegenwart eines Katalysators, wie beispielsweise Natriumwolframat, durchzuführen.

Geeignete Lösungsmittel sind halogenierte Kohlenwasserstoffe wie Methylenchlorid, Chloroform und Chlorbenzol, Alkohole wie Methanol, Ethanol, n-Propanol, Isopropanol, n-Butanol und tert.-Butanol.

Die Edukte werden im allgemeinen in äquimolaren Mengen miteinander umgesetzt. Es kann vorteilhaft sein, das Oxidationsmittel in einem Überschuß bezogen auf VI einzusetzen.

Anschließend setzt man die Verbindungen der Formel II mit einem Thiophenderivat der Formel III zu N-heterocyclylsubstituierten Thienyloxy-Pyrimidinen der Formel I um:

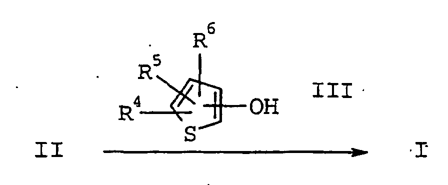

Diese Umsetzung erfolgt üblicherweise bei Temperaturen von 0 °C bis 130 °C, vorzugsweise 25 °C bis 40 °C, in einem inerten organischen Lösungsmittel in Gegenwart einer Base [vgl. WO 98/40379]

Geeignete Lösungsmittel sind Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol, sowie Dimethylsulfoxid und Dimethylformamid, besonders bevorzugt Dimethylformamid.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie

Alkalimetallhydrogencarbonate wie Natriumhydrogencarbonat, in Betracht. Besonders bevorzugt werden Alkalimetall- und Erdalkalimetallcarbonate.

Die Basen werden im allgemeinen in äquimolaren Mengen eingesetzt.

Die Edukte werden im allgemeinen in äquimolaren Mengen miteinander umgesetzt.

'Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid, Essigsäureethylester extrahiert. Der organische Extrakt kann mit 5-10%iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt.

### Verfahren B

Dichloroheterocyclen der Formel VIII werden mit Thiophenderivaten der Formel III zu Thienyloxy-Pyrimidinderivaten der Formel IV umgesetzt:

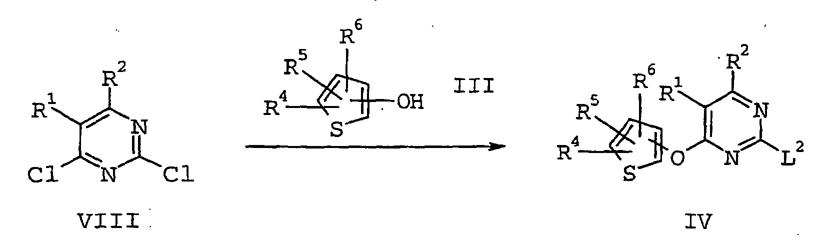

Diese Umsetzung erfolgt üblicherweise bei Bedingungen wie sie zuvor bei der Umsetzung von II zu I beschrieben wurden.

L² steht für eine Abgangsgruppe wie Halogen, z.B. Chlor, Brom oder Iod, C₁-C₄-Alkylsulfonyl, C₁-C₄-Halogenalkylsulfonyloxy oder Trialkylammonium, bevorzugt sind Chlor, C₁-C₄-Alkylsulfonyl, wie z.B. Methylsulfonyl, oder C₁-C₄-Halogenalkylsulfonyloxy, wie z.B. Trifluormethylsulfonyloxy.

Anschließend setzt man die Thienyloxy-Pyrimidinderivate der Formel IV mit Azolen der Formel V zu N-heterocyclylsubstituierten Thienyloxy-Pyrimidinen der Formel I um:

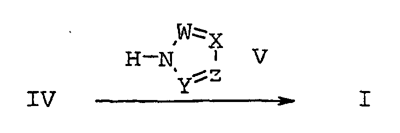

Diese Umsetzung erfolgt in Gegenwart einer Base üblicherweise bei Bedingungen wie sie zuvor bei der Umsetzung von VII zu VI beschrieben wurden.

Beispielsweise kann die Umsetzung wie folgt durchgeführt werden:
Bei Pyrazolen und Imidazolen in Gegenwart einer Base wie z.B. Kaliumcarbonat in Dimethylformamid;
bei Pyrrolen in Gegenwart einer Base wie z.B. Kalium-tert.butylat und DABCO in Tetrahydrofuran;
bei Trianolen in Gegenwart einer Base wie z.B. Kaliumcarbonat und DABCO in Acetonitril.

Daneben kann diese Umsetzung auch palladiumkatalysiert erfolgen. Dann erfolgt die Umsetzung üblicherweise bei Temperaturen von 25 °C bis 130 °C in einem inerten organischen Lösungsmittel in Gegenwart einer Base [vgl. J. F. Hartwig et al., J. Am. Chem. Soc. 1998, 120, 827-828; S. L. Buchwald et al., J. Organomet. Chem. 1999, 576, 125-146].

Als Katalysatoren eignen sich z.B. Palladiumligandkomplexe, in denen das Palladium in der Oxidationsstufe 0 vorliegt, metallisches Palladium, das gegebenenfalls auf einen Träger aufgezogen wurde und vorzugsweise Palladium(II)salze. Die Umsetzung mit Palladium(II)salzen und metallischem Palladium wird vorzugsweise in Gegenwart von Komplexliganden durchgeführt.

Als Palladium(0)komplexligand kommen beispielsweise Tetrakis(triphenylphosphan)palladium, Palladium(diphenylphosphinoferrocen)dichlorid {[PdCl₂(dppf)]} oder Tris(dibenzylidenaceton)dipalladium Pd₂(dba)₃ in Frage.

Als Palladium(II) salze eigenen sich beispielsweise Palladiumacetat und Palladiumchlorid. Bevorzugt wird in Gegenwart von Komplexliganden wie beispielsweise Triphenylphosphan gearbeitet.

Die Herstellung der komplexen Palladiumsalze kann in an sich bekannter Weise ausgehend von kommerziell erhältlichen Palladiumsalzen wie Palladiumdichlorid oder Palladiumdiacetat und den entsprechenden Phosphanen wie z.B. Triphenylphosphan oder 1,2-Bis(diphenylphosphano)ethan erfolgen. Ein Großteil der komplexierten Palladiumsalze ist auch kommerziell erhältlich. Bevorzugte Palladiumsalze sind [(R)(+)2,2'-Bis(diphenylphosphano)-1,1'-binaphthyl]palladium(II)chlorid, Bis(triphenylphosphan)palladium(II)acetat und insbesondere Bis(triphenylphosphan)palladium(II)chlorid.

Der Palladiumkatalysator wird in der Regel in einer Konzentration von 0,05 bis 5 Mol%, bevorzugt 1-3 Mol%, eingesetzt.

Geeignete Lösungsmittel sind aromatische Kohlenwasserstoffe wie Toluol, o-, m- und p-Xylol, Ether wie Diethylether, Diisopropylether, tert.-Butylmethylether, Dioxan, Anisol und Tetrahydrofuran, sowie Dimethylformamid.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat sowie Alkalimetall- und Erdalkalimetallalkoholate wie Natriummethanolat, Natriumethanolat, Kaliumethanolat, Kalium-tert.-butylat und Dimethoxymagnesium.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, V in einem Überschuß bezogen auf IV einzusetzen.

Die Aufarbeitung kann in an sich bekannter Weise zum Produkt hin erfolgen.

### Verfahren C

Es ist ebenso möglich den Stickstoffheterocyclus direkt aus einem entsprechenden Aminopyrimidin aufzubauen. Man erhält dann N-heterocyclylsubstituierte Pyrimidine, welche anschließend gemäß der zuvor dargestellten Reaktionen weiter modifiziert werden können. Diese Variante C wird exemplarisch an einem Aminopyrimidin der

Formel XIV dargestellt, aus dessen Umsetzung man N-heterocyclylsubstituierte Pyrimidine der Formel XV erhält. Der Aufbau des Heterocyclus kann aber auf einer anderen Stufe der oben dargestellten Varianten A oder B erfolgen.

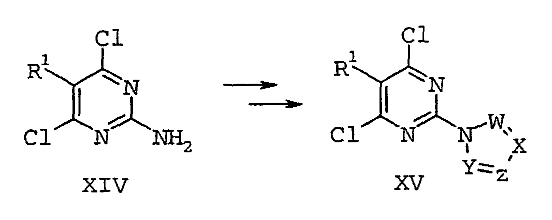

Die nachstehend genannten Reaktionen sind literaturbekannt und werden unter anderem beschrieben in T. Eicher, S. Hauptmann, The Chemistry of Heterocycles; J. A. Joule, K. Mills, Heterocyclic Chemistry.

Pyrrolderivate können dargestellt werden, indem man in einer Paal-Knorr-Synthese das entsprechende primäre Amin mit einer Dicarbonylverbindung umsetzt. Über α-Halogencarbonylverbindungen gelangt man mit β-Ketoestern und primären Aminen zu 3-Alkoxycarbonyl- oder 3-Acyl-substituierten Pyrrolen (Hantzsch-Synthese). Pyrimidyl-substituierte Pyrrole erhält man durch die Reaktion von aliphatischen oder aromatischen Aminen mit Dimethoxytetrahydrofuran oder 1,4-Dichloro-1,4-dimethoxybutan.

Imidazole erhält man durch Umsetzung von Isocyanaten mit Iminen unter basischen Bedingungen oder durch die Reaktion von 2-Bromketonen mit Amidin- oder Guanidinderivaten unter dem Einfluß einer Base. Die entsprechenden Amidin- oder Guanidinderivate werden in Analogie zu literaturbekannten Verfahren aus XIV hergestellt.

Pyrazole können synthetisiert werden, indem man das primäre Amin zunächst in die Diazoniumverbindung überführt. Nach Hydrierung erhält man das entsprechende Pyrimidinhydrazin-Derivat, das mit 1,3-Dicarbonylverbindungen, Enolestern oder 1-Alkinylketonen in einer Cyclokondensation zum gewünschten Pyrazol führt.

1,2,3-Triazole können durch die Umsetzung von Aziden mit Alkinen oder CH-aciden Verbindungen in Anwesendheit eines Alkoholats erhalten werden. Die Kondensation von Aziden mit Acyl-Wittig-Reagenzien bietet eine Möglichkeit einer regiospezifischen Synthese von 1,5-disubstituierten 1,2,3-Triazolen.

1,2,4-Triazole erhält man durch Umsetzung von Pyrimidinhydrazin-Derivaten mit Diacylaminen in Anwesendheit schwacher Säuren oder durch die Umsetzung von N,N'-Diacylhydrazin mit Aminopyridin-Derivaten unter dem Einfluß von Phosphorpentoxid.

Tetrazole können synthetisiert werden durch [3+2]Cycloaddition von Aziden mit Nitrilen oder einem aktivierten Amid. Die Reaktion von Arylthioisocyanaten mit Aziden oder Nitrosierung von Pyrimidylammoniumhydrazonen sind alternative Methoden für die Synthese von substituierten Tetrazolen.

Thienyloxy-Pyrimidinderivate der Formel IV 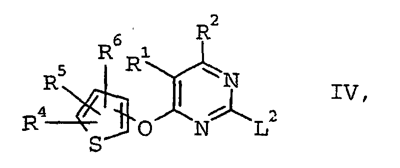 wobei R¹, R², R⁴, R⁵ und R⁶ die unter Anspruch 1 genannten Bedeutungen haben und L² für eine nucleophil austauschbare Abgangsgruppe wie Halogen, z.B. Chlor, Brom oder Iod, C₁-C₄-Alkylsulfonyl, C₁-C₄-Halogenalkylsulfonyloxy oder Trialkylammonium steht, bevorzugt sind Chlor, C₁-C₄-Alkylsulfonyl wie z.B. Methylsulfonyl, oder C₁-C₄-Halogenalkylsulfonyloxy, wie z.B. Trifluormethylsulfonyloxy, sind Schlüsselzwischenprodukte bei der Synthese der erfindungsgemäßen Triphenyloxy-Pyrimidinderivate der Formel I gemäß Verfahren B.

Die besonders bevorzugten Ausführungsformen der Zwischenprodukte in Bezug auf die Variablen entsprechen denen der Reste R¹, R², R⁴, R⁵ und R⁶ der Formel I.

Besonders bevorzugt werden Zwischenprodukte der Formel IV, in denen
- R¹: Wasserstoff, Halogen, Cyano, C₁-C₆-Alkyl, C₁-C₆-Halogenalkyl oder C₁-C₆-Alkoxy;
besonders bevorzugt Wasserstoff, Halogen oder C₁-C₆-Alkyl; insbesondere bevorzugt Wasserstoff, Fluor, Chlor oder Methyl;
- R²: Wasserstoff, Halogen, Cyano, C₁-C₆-Alkyl, C₁-C₆-Halogenalkyl, C₁-C₆-Alkoxy, C₁-C₆-Halogenalkoxy, C₁-C₆-Alkoxy-C₁-C₄-alkyl, C₁-C₆-Alkylthio oder COOR⁷; besonders bevorzugt Wasserstoff, Halogen, Cyano, C₁-C₆-Alkyl, C₁-C₆-Halogenalkyl, C₁-C₆-Alkoxy, C₁-C₆-Alkoxy-C₁-C₄-alkyl oder C₁-C₆-Alkylthio; insbesondere bevorzugt Wasserstoff, Halogen, C₁-C₆-Alkyl oder C₁-C₆-Halogenalkyl; sehr bevorzugt Wasserstoff, Fluor, Chlor, Methyl oder Triflourmethyl;
- R⁴, R⁵, R⁶: Wasserstoff, Halogen, Cyano, C₁-C₆-Alkyl, C₁-C₆-Halogenalkyl C₁-C₆-Alkoxy, C₁-C₆-Halogenalkoxy, C₁-C₆-Alkylthio, C₁-C₆-Alkylsulfonyl oder C₁-C₆-Halogenalkylsufonyl;
besonders bevorzugt Wasserstoff, Halogen, Cyano, C₁-C₆-Alkyl, C₁-C₆-Halogenalkyl, C₁-C₆-Alkoxy, C₁-C₆-Alkylsulfonyl oder C₁-C₆-Halogenalkylsufonyl; insbesondere bevorzugt Wasserstoff, Halogen, C₁-C₆-Halogenalkyl, C₁-C₆-Alkylsulfonyl oder C₁-C₆-Halogenalkyl-sufonyl;
sehr bevorzugt Wasserstoff, Fluor, Chlor, Trifluormethyl oder Methylsulfonyl
bedeuten.

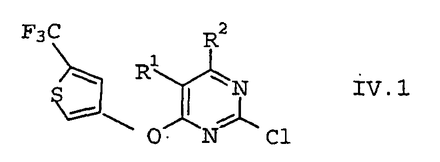

**Tabelle 3**

| Nr. | R¹ | R² | ¹H-NMR/CDCl₃ bzw. Schmp. [°C] |
|---|---|---|---|
| IV.1.1 | H | CH₃ | δ= 2,53 (s,3H), 6,71 (s, 1H), 7,35 (1H, J = 1,77 Hz) |
| IV.1.2 | CH₃ | H | δ= 2,29 (s, 3H), 7,39 (m, 1H), 7,45 (1H, J = 1,77 Hz), 8,31 (m, 1H). |
| IV.1.3 | H | H | δ= 2,43 (3H,s), 6,58 (1H,d) 7,32 (1H, d), 7,38 (1H,m), 8,4 (1H,d). |
| IV.I.4 | CH₃ | CH₃ | δ= 2,25 (3H,s), 2,50 (3H,s), 7,34 (1H,s), 7,40 (1H,s). |

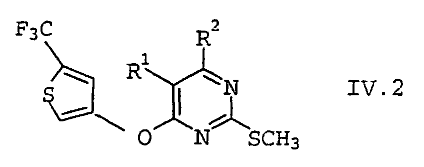

**Tabelle 4**

| Nr. | R¹ | R² | ^{1H}-NMR/CDCl₃ bzw. Schmp. [°C] |
|---|---|---|---|
| IV.2.1 | H | Cl | δ= 2,43 (3H,s), 6,60 (1H,s), 7,30 (1H,d) 7,35 (1H,m). |
| IV.2.2 | H | OCH₃ | δ= 2,43 (3H,s), 3,96 (3H,s), 6,86 (1H,s) 7,24 (1H,d), 7,35 (1H,m). |
| IV.2.3 | H | CF₃ | δ= 2,48 (3H,s), 6,90 (1H,s), 7,37 (1H,d) 7,38 (1H,m). |

### Herstellungsbeispiele:

### 5-Methyl-2 [3-trifluormethyl)-1H-pyrazol-1-yl]-4-{[5- (trifluormethyl)-3-thienyl]oxy}pyrimidin

### Verfahren A:

### 2,4-Dichlor-5-anethylpyrimidin

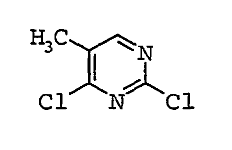

Zu einer Lösung von 60 ml Phosphoroxychlorid wurden bei Raumtemperatur langsam 2 ml N,N-Dimethylformamid zugegeben. Die Mischung wurde 10 min gerührt, dann 10 g (79.3 mmol) Thymin portionsweise zugegeben und weitere 10 min gerührt. Dann wurde die Mischung langsam auf 105 °C erwärmt und 3 h bei dieser Temperatur gerührt. Es wurde mit 800 ml H₂O hydrolysiert und die Lösung mit Essigsäureethylester extrahiert. Die abgetrennten vereinigten organischen Phasen wurden mit H₂O gewaschen, getrocknet und vom Lösungsmittel befreit. Man erhielt 12.3 g (95 % der Theorie) der Titelverbindung.
1H NMR (400 MHz, CDCl₃) δ = 2.35 (s, 3H), 8.45 (s, 1H) 2-Chlor-5-methyl-4-(methylthio)pyrimidin

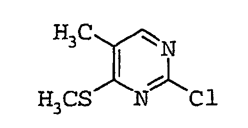

Zu einer Lösung von 13.77 g (84.5 mmol) 2,4-Dichlor-5-methylpyrimidin in 100 ml Tetrahydrofuran wurden unter Eiskühlung 7.3 g (98.02.mmol) NaSCH₃ portionsweise zugegeben. Die Reaktionsmischung wurde 6 h bei Raumtemperatur gerührt. Dann wurde mit H₂O hydrolysiert und die Reaktionsmischung mit Essigsäureethylester extrahiert. Die vereinigten organischen Phasen wurden mit H₂O gewaschen, getrocknet und das Lösungsmittel destillativ entfernt. Man erhielt 13.32 g (90 % der Theorie) der Titelverbindung als farblosen Feststoff.
Schmp.: 73-76 °C
1H NMR (400 MHz, CDCl₃) δ = 2.15 (s, 3H), 2.60 (s, 3H), 8.01 (s, 1H).

5-Methyl-4-methylthio-2-[3-(trifluormethyl)-1H-pyrazol-1-yl]-pyrimidin

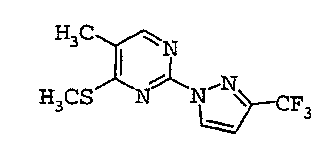

Eine Mischung von 12.32 g (70.5 mmol)2-Chlor-5-methyl-4-(methylthio)pyrimidin, 11.5 g (84.6 mmol)3-Trifluoromethyl-1H-pyrazol, 24.3 g (176.4 mmol) Natriumcarbonat und 150 ml N,N-Dimethylformamid wurde 6 h auf 100 °C erhitzt und anschließend bei 25 °C über Nacht gerührt. Anschließend wurden die Phasen getrennt, die organische Phase wurde gewaschen, getrocknet und vom Lösungsmittel befreit. Man erhielt 18.15 g (94 % der Theorie) der Titelverbindung.
1H NMR (400 MHz, CDCl₃) δ = 2.25 (s,3H), 2.65 (s, 3H), 6,73 (s, 1H), 8.23 (s, 1H), 8.63 (s, 1H). 2-(4,5-Dichlor-1H-imidazol-1-yl)-5-methyl-4-(methylthio)-pyrimidin

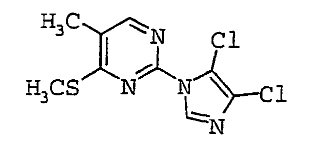

Eine Mischung von 8.22 g (46.9 mmol)2-Chlor-5-methyl-4-(methylthio)pyrimidin, 8.5 g (61.8 mmol)4,5-Dichloro-1H-imidazol, 17.8 g (128.8 mmol) Natriumcarbonat und 150 ml N,N-Dimethylformamid wurde 12 h auf 100 °C erhitzt. Anschließend wurden die Phasen getrennt, die organische Phase wurde gewaschen, getrocknet und vom Lösungsmittel befreit. Man erhielt 18.15 g (94 % der Theorie) der Titelverbindung.
1H NMR (400 MHz, CDCl₃) δ = 2.25 (s, 3H), 2.62 (s, 3H), 8.15 (s, 1H), 8.35 (s, 1H) .

5-Methyl -4-methylsulfon-2- [3-(trifluoromethyl) -1H-pyrazol-1-yl]-pyrimidin

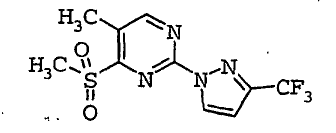

Zu einer Lösung von 16 g (58.3 mmol) von 5-Methyl-4-methylthio-2-[3-(trifluoromethyl)-1H-pyrazol-1-yl]-4-pyrimidin in 150 ml Methylenchlorid wurden 33.6 g (116.7 mmol) 60%ige Metachlorper-benzoesäure gegeben. Die Reaktionsmischung wurde 4 h bei 25 °C gerührt und anschließend filtriert. Das Filtrat wurde mit Natriumhydrogensulfid, Natriumhydrogencarbonat und H₂O gewaschen und getrocknet. Das Lösungsmittel wurde destillativ entfernt. Man erhielt 16 g (90 % der Theorie) der Titelverbindung als farblosen Feststoff.
Schmp.: 107-111 °C
1H NMR (400 MHz, CDCl₃) δ = 2.72 (s, 3H), 3.47 (s, 3H), 6.79 (s, 1H); 8.58 (s, 1H), 8.85 (s, 1H).

5-Methyl-2[3-trifluormethyl)-1H-pyrazol-1-yl]-4-{[5-(trifluormethyl)-3-thienyl]oxy}pyrimidin

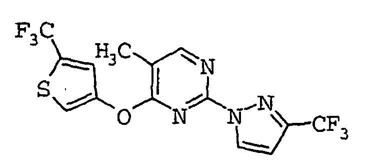

Eine Mischung von 1 g (3.27 mmol) 5-Methyl-4-methylsulfon-2-[3-(trifluormethyl)-1H-pyrazol-1-yl]-4-pyrimidin, 0.71 g (4.24 mmol) 5-(Trifluormethyl)-3-hydroxy-Thien und 0.9 g (6.53 mmol) Natriumcarbonat wurde in 20 ml N,N-Dimethylformamid bei 25 °C 2 h gerührt. Die Phasen wurden getrennt und die organische Phase wurde gewaschen, getrocknet und vom Lösungsmittel befreit. Das Rohprodukt wurde chromatographisch gereinigt (Cyclohexan/Essigsäureethylester 10 : 1) . Man erhielt 0.7 g (54 % der Theorie) der Titelverbindung als farblosen Feststoff.
Schmp.: 113-115 °C.
1H NMR (400 MHz, CDCl₃) δ = 2.35 (s, 3H), 6.70 (s, 1H), 7.48 (s, 2H), 8.28 (s,1H), 8.52 (s, 1H).

In Tabellen 5 und 6 sind neben der voranstehenden Verbindung noch weitere N-heterocyclylsubstituierte Pyrimidine der Formel I aufgeführt, die in analoger Weise hergestellt wurden oder herstellbar sind.

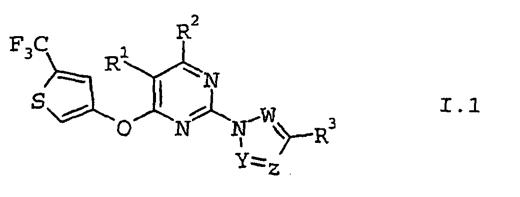

**Tabelle 5**

| Nr. | R¹ | R² | R³ | W | Y | Z | Schmp.[°C] bzw. ¹H-NMR/CDCl₃ |
|---|---|---|---|---|---|---|---|
| I.1.1 | CH₃ | H | CF₃ | N | CH | CH | 113-115 |
| I.1.2 | CH₃ | H | CF₃ | CH | CH | N | |
| I.1.3 | CH₃ | H | CF₃ | N | CH | N | |
| I.1.4 | CH₃ | H | Cl | CCl | CH | N | 113-115 |
| I.1.5 | CH₃ | H | H | CNO₂ | CCH₃ | N | 126-129 |
| I.1.6 | CH₃ | H | C(CH₃)₃ | N | CBr | N | δ= 1,40(9H,s),2,36 (3Hs),7,36(1H,s), 7,44(1H,s),8,64 (1H,s). |
| I.1.7 | CH₃ | H | Br | N | CNO₂ | N | |
| I.1.8 | H | CH₃ | CF₃ | N | CH | CH | |
| I.1.9 | H | CH₃ | CF₃ | CH | CH | N | |
| I.1.10 | H | CH₃ | CF₃ | N | CH | N | |
| I.1.11 | H | CH₃ | Cl | CCl | CH | N | 97-99 |
| I.1.12 | H | CH₃ | H | CNO₂ | CCH₃ | N | |
| I.1.13 | H | CH₃ | C(CH₃)₃ | N | CBr | N | |
| I.1.14 | H | CH₃ | Br | N | CNO₂ | N | |
| I.1.15 | H | H | CF₃ | N | CH | CH | 10-111 |
| I.1.16 | H | OCH₃ | CF₃ | N | CH | CH | 74-79 |
| I.1.17 | H | CF₃ | CF₃ | N | CH | CH | 164-169 |
| I.1.18 | CH₃ | CH₃ | CF₃ | N | CH | CH | 105-116 |
| I.1.19 | H | H | Cl | CCl | CH | N | δ= 6,96(1H,d),7,34 (1H,d),7,41(1H,m), 8, 31 (1H,s), 8, 63 (1H,d). |
| I.1.20 | CH₃ | CH₃ | Cl | CCl | CH | N | δ= 2,17(3H,s),2,63 (3H,s),7,38(1H,d), 7,40(1H,m),7,57 (1H,s). |
| I.1.21 | CH₃ | H | NO₂ | CH | CCH₃ | N | 126-129 |
| I.1.22 | CH₃ | CH₃ | NO₂ | CH | CCH₃ | N | δ= 2,34 (3H,s),2,47 (3H,s),2,58(3H,s) 7,21(1H,d),7,33 (1H,m),8,55(1H,s) . |
| I.1.23 | H | CH₃ | NO₂ | CH | CCH₃ | N | δ= 2,53(3H,s),2,60 (3H,s),6,81(1H,s), 7,23(1H,d),7,33 (1H,m),8,60(1H,s). |
| I.1.24 | H | OCH₃ | C(CH₃)₃ | N | CBr | N | 126-127 |
| I.1.25 | H | CH₃ | S-*n*-C₆H₁₄ | N | CH | N | δ= 0,90(3H,t),1,30-1,48(6H,m),1,56 (3H,s)1,76-1,81 (2H,m),2,58(3H,s), 3,20(2H,t),7,34 1H,d),7,42(1H,s), 8,94(1H,s). |
| I.1.26 | CH₃ | H | S-n-C₆H₁₄ | N | CH | N | δ= 0,89(3H,t),1,30-1,48(6H,m),1,56( (3H,s),1,76-1,81 (2H,m),2,63(3H,s), 3,19(2H,t),7,31 (1H,d),7,41(1H,m), 8,51(1H,s),9,01 (1H,s). |
| I.1.27 | CH₃ | H | CF₃ | N | N | CH | δ= 2,42(3H,d),7,46 (1H,m),7,71(1H,d), 8,56(1H,d),8,64 (1H,d). |
| I.1.28 | H | H | CF₃ | N | N | CH | δ= 7,08(1H,d),7,44 (1H,m) ,7, 69 (1H,d) 8,71(1H,s),8,75 (1H,d). |
| I.1.29 | CH₃ | H | CF₃ | CH | N | N | δ= 7,57(1H,m),7,91 (1H,d),8,15(1H,s), 8,59(1H,d). |
| I.1.30 | H | H | CF₃ | CH | N | N | δ= 7,06(1H,d),7,53 (1H,m),7,83(1H,d), 8,19(1H,s),8,79 (1H,d). |

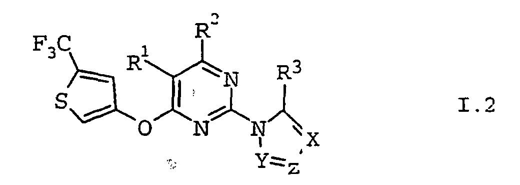

**Tabelle 6**

| Nr. | R¹ | R² | R³ | X | Y | Z | Schmp. [°C] bzw. ¹H-NMR/CDCl₃ |
|---|---|---|---|---|---|---|---|
| I.2.1 | H | CH₃ | CH₃ | N | CH | N | 172-178 |
| I.2.2 | CH₃ | H | CH₃ | N | CH | N | 85-87 |
| I.2.3 | CH₃ | CH₃ | CH₃ | N | CH | N | δ= 2,50(3H,s),2,55 (3H,s),2,60(3H,s), 7,35(1H,s),7,42 (1H,s),8,82(1H,s). |
| I.2.4 | CH₃ | H | Cl | N | CCl | N | δ= 2,40(3H,s),7,40 (1H,s)7,48(1H,s), 8,32(1H,s). |
| I.2.5 | CH₃ | H | CF₃ | N | CH | N | 73-75 |
| I.2.6 | H | CH₃ | CF₃ | N | CH | N | δ= 2,63(3H,s),7,36 (1H,d),7,44(1H,m), 7,57(1H,s),9,12 (1H,s) |
| I.2.7 | H | H | CF₃ | N | CH | N | δ= 7,04(1H,d),7,45 (1H,m),7,50(1H,d), 8,74(1H,d),9,05 (1H,s) |
| I.2.8 | CH₃ | H | CH₃ | CH | CCH₃ | CH | δ=1,56(6H,s),2,16 (3H,s),7,18(1H,d) 7,25 (1H,d),7,30 (1H,d) ,7,32(1H,m), 8,26(1H,s) |
| I.2.9 | H | CH₃ | CN | CH | CH | CH | 98-100 |
| I.2.10 | CH₃ | H | CN | CH | CH | CH | 130-136 |
| I.2.11 | H | H | C(=O)CF₃ | CH | CH | CH | δ= 6,52 (1H,m),7,05 (1H,d),7,32(1H,s), 7,30(1H,s),7,48 (1H,m),7,62(1H,m) 8,67(1H,d) |
| I.2.12 | CH₃ | H | C(=O)CF₃ | CH | CH | CH | δ= 2,30(3H,s),7,32 1H,m),7,15-7,25 (2H,m),7,40(1H,s) 7,45(1H,s)8,34 (1H,s). |

### Biologische Wirkamkeit

Die N-heterocyclylsubstituierten Thienyloxy-Pyrimidine der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im Wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile eines Wirkstoffs der Formel I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile eines Wirkstoffs der Formel I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile eines Wirkstoffs der Formel I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile eines wirkstoffs der Formel I werden mit 3 Gewichtsteilen des Natriumsalzes der Diisobutylnaphthalin-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile eines Wirkstoffs der Formel I werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile eines Wirkstoffs der Formel I werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil eines Wirkstoffs der Formel I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil eines Wirkstoffs der Formel I wird in einer Mischung gelöst, die aus 80 Gewichtsteilen Cyclohexanon und 20 Gewichtsteilen Wettol^{R} EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0.001 bis 3,0, vorzugsweise 0,01 bis 1,0 kg/ha aktive Substanz (a.S.).

Zur Verbreiterung des Wirkungsspektrums und zur Erzielung synergistischer Effekte können die N-heterocyclylsubstituierten Thienyloxy-Pyrimidine der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der N-heterocyclylsubstituierten Thienyloxy-Pyrimidine der Formel I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,25 bzw. 0,125 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Auf gang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Amaranthus retroflexus | Fuchsschwanz | pig weed |
| Chenopodium album | Weißer Gänsefuß | lambsquaters |
| Galium aparine | Klettenabkraut | catchweed |
| Pharbitis purpurea | Trichterwinde | tallmorningglory |
| Polygonum persicaria | Flohknöterich | ladysthumb |

Bei Aufwandmengen von 0,25 bzw. 0,125 kg/ha zeigte die Verbindung I.1.1 (Tabelle 5) im Nachauflauf eine sehr gute Wirkung gegen die unerwünschten Pflanzen Amaranthus retroflexus, Chenopodium album, Galium aparine, Pharbitis purpurea und Polygonum persicaria.

## Patentansprüche

1. N-heterocyclylsubstituierte Thienyloxy-Pyrimidine der Formel I 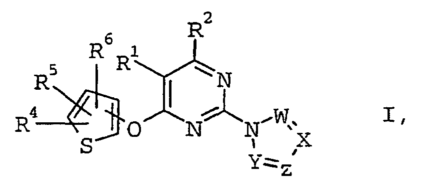 in der die Variablen die folgenden Bedeutungen haben:
W, X, Y, Z unabhängig voneinander N oder CR³, wobei mindestens eine der Variablen CR³ bedeutet
R¹ Wasserstoff, Halogen, Cyano, C₁-C₆-Alkyl, C₁-C₆-Halogenalkyl, C₁-C₆-Alkoxy oder C₁-C₆-Halogenalkoxy;
R² Wasserstoff, Halogen, Cyano, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₆-Alkoxy, C₃-C₆-Alkenyloxy, C₃-C₆-Alkinyloxy, C₁-C₆-Halogenalkoxy, C₁-C₆-Alkoxy-C₁-C₄-alkyl, C₁-C₆-Alkylamino, Di-(C₁-C₄-alkyl)amino, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, COOR⁷ oder CONR⁸R⁹;
R³ Wasserstoff, Halogen, Cyano, Nitro, C₁-C₆-Alkyl, C₁-C₆-Halogenalkyl, C₁-C₆-Alkoxy, C₁-C₆-Halogenalkoxy, C₁-C₆-Alklylthio, C₁-C₆-Halogenalkylthio, C₁-C₆-Alkylsulfonyl oder COOR⁷;
R⁴, R⁵, R⁶ Wasserstoff, Halogen, Cyano, C₁-C₆-Alkyl, C₁-C₆-Halogenalkyl, C₁-C₆-Alkoxy, C₁-C₆-Halogenalkoxy, C₁-C₆-Alklylthio, C₁-C₆-Halogenalkylthio, C₁-C₆-Alkylsulfonyl oder C₁-C₆-Halogenalkylsulfonyl;
R⁷ Wasserstoff, C₁-C₄-Alkyl, C₃-C₄-Alkenyl, C₃-C₄-Alkinyl oder C₁-C₄-Halogenalkyl;
R⁸ Wasserstoff, C₁-C₄-Alkyl, C₃-C₄-Alkenyl, C₃-C₄-Alkinyl oder C₁-C₄-Alkoxy;
R⁹ Wasserstoff, C₁-C₄-Alkyl, C₃-C₄-Alkenyl oder C₃-C₄-Alkinyl;
sowie deren landwirtschaftlich brauchbaren Salze.

2. N-heterocyclylsubstituierte Thienyloxy-Pyrimidine der Formel I nach Anspruch 1, in der die Variablen
W, X, Y, Z unabhängig voneinander N oder CR³ sind, wobei maximal eine der Variablen N bedeutet.

3. N-heterocyclylsubstituierte Thienyloxy-Pyrimidine der Formel I nach Anspruch 1 oder 2, in der
R¹ Wasserstoff, Halogen, Cyano, C₁-C₆-Alkyl, C₁-C₆-Halogenalkyl oder C₁-C₆-Alkoxy;
R² Wasserstoff, Halogen, Cyano, C₁-C₆-Alkyl, C₁-C₆-Halogenalkyl, C₁-C₆-Alkoxy, C₁-C₆-Halogenalkoxy, C₁-C₆-Alkylthio oder C₁-C₆-Halogenalkylthio
bedeuten.

4. N-heterocyclylsubstituierte Thienyloxy-Pyrimidine der Formel I nach Anspruch 1 bis 3, in der
R³ Wasserstoff, Halogen, Cyano, C₁-C₆-Alkyl, C₁-C₆-Halogenalkyl oder C₁-C₆-Alkoxy
bedeutet.

5. N-heterocyclylsubstituierte Thienyloxy-Pyrimidine der Formel I nach Anspruch 1 bis 4, in der
R⁴, R⁵, R⁶ Wasserstoff, Halogen, Cyano, C₁-C₆-Alkyl, C₁-C₆-Halogenalkyl oder C₁-C₆-Halogenalkoxy
bedeuten.

6. Verfahren zur Herstellung von N-heterocyclylsubstituierten Thienyloxy-Pyrimidinen der Formel I gemäß Anspruch 1, **dadurch gekennzeichnet, daß** man Pyrimidine der Formel II 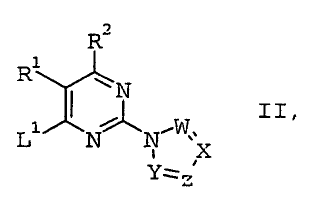 wobei W, X, Y, Z, R¹ und R² die unter Anspruch 1 genannten Bedeutungen haben und L¹ für eine nucleophil austauschbare Abgangsgruppe steht,
mit einem Thiophenderivat der Formel III 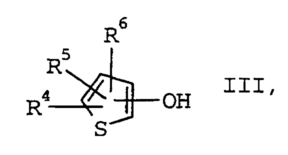 wobei R⁴, R⁵ und R⁶ die unter Anspruch 1. genannten Bedeutungen haben, umsetzt.

7. Verfahren zur Herstellung von N-heterocyclylsubstituierten Thienyloxy-Pyrimidinen der Formel I gemäß Anspruch 1, **dadurch gekennzeichnet, daß** man Thienyloxy-Pyrimidinderivate der Formel IV 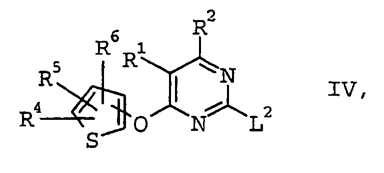 wobei R¹, R², R4, R⁵ und R⁶ die unter Anspruch 1 genannten Bedeutungen haben und L² für eine nucleophil austauschbare Abgangsgruppe steht,
. mit einem Stickstoff-Heterocyclus der Formel V 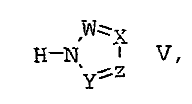 wobei W, X, Y und Z die unter Anspruch 1 genannten Bedeutungen haben, umsetzt.

8. Thienyloxy-Pyrimidinderivate der Formel IV 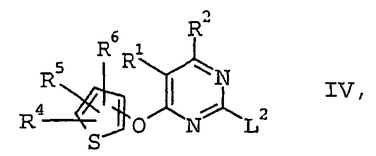 wobei R¹, R², R⁴, R⁵ und R⁶ die unter Anspruch 1 genannten Bedeutungen haben und L² für eine nucleophil austauschbare Abgangsgruppe steht.

9. Mittel, enthaltend eine herbizid wirksame Menge mindestens eines N-heterocyclylsubstituierten Thienyloxy-Pyrimidins der Formel I oder eines landwirtschaftlich brauchbaren Salzes von I gemäß den Ansprüchen 1 bis 5 und für die Formulierung von Pflanzenschutzmitteln übliche Hilfsmittel.

10. Verfahren zur Herstellung von Mitteln gemäß Anspruch 9, **dadurch gekennzeichnet, daß** man eine herbizid wirksame Menge mindestens eines N-heterocyclylsubstituierten Thienyloxy-Pyrimidinderivates der Formel I gemäß den Ansprüchen 1 bis 5 oder eines landwirtschaftlich brauchbaren Salzes von I und für die Formulierung von Pflanzenschutzmitteln übliche Hilfsmittel mischt.

11. Verfahren zur Bekämpfung von unerwünschtem Pflanzenwuchs, **dadurch gekennzeichnet, daß** man eine herbizid wirksame Menge mindestens eines N-heterocyclylsubstituierten Thienyloxy-Pyrimidinderivates der Formel I gemäß den Ansprüchen 1 bis 5 oder eines landwirtschaftlich brauchbaren Salzes von I auf Pflanzen, deren Lebensraum und/oder auf Samen einwirken läßt.

12. Verwendung der N-heterocyclylsubstituierten Thienyloxy-Pyrimidinderivate der Formel I gemäß den Ansprüchen 1 bis 5 und deren landwirtschaftlich brauchbaren Salze als Herbizide.

## Claims

1. An N-heterocyclyl-substituted thienyloxypyrimidine of the formula I 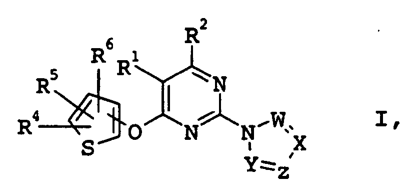 where:
W, X, Y, Z independently of one another are N or CR³, where at least one of the variables is CR³;
R¹ is hydrogen, halogen, cyano, C₁-C₆-alkyl, C₁-C₆-haloalkyl, C₁-C₆-alkoxy or C₁-C₆-haloalkoxy;
R² is hydrogen, halogen, cyano, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₁-C₆-alkoxy, C₃-C₆-alkenyloxy, C₃-C₆-alkynyloxy, C₁-C₆-haloalkoxy, C₁-C₆-alkoxy-C₁-C₄-alkyl, C₁-C₆-alkylamino, di-(C₁-C₄-alkyl)amino, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, COOR⁷ or CONR⁸R⁹;
R³ is hydrogen, halogen, cyano, nitro, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfonyl or COOR⁷;
R⁴, R⁵, R⁶ are hydrogen, halogen, cyano, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfonyl or C₁-C₆-haloalkylsulfonyl;
R⁷ is hydrogen, C₁-C₄-alkyl, C₃-C₄-alkenyl, C₃-C₄-alkynyl or C₁-C₄-haloalkyl;
R⁸ is hydrogen, C₁-C₄-alkyl, C₃-C₄-alkenyl, C₃-C₄-alkynyl or C₁-C₄-alkoxy;
R⁹ is hydrogen, C₁-C₄-alkyl, C₃-C₄-alkenyl or C₃-C₄-alkynyl;
and its agriculturally useful salts.

2. An N-heterocyclyl-substituted thienyloxypyrimidine of the formula I as claimed in claim 1 where
W, X, Y, Z independently of one another are N or CR³, where at most one of the variables is N.

3. An N-heterocyclyl-substituted thienyloxypyrimidine of the formula I as claimed in claim 1 or 2 where
R¹ is hydrogen, halogen, cyano, C₁-C₆-alkyl, C₁-C₆-haloalkyl or C₁-C₆-alkoxy;
R² is hydrogen, halogen, cyano, C₁-C₆-alkyl, C₁-C₆-haloalkyl, C₁-C₆-alkoxy, C₁-C₆-haloalkoxy, C₁-C₆-alkylthio or C₁-C₆-haloalkylthio.

4. An N-heterocyclyl-substituted thienyloxypyrimidine of the formula I as claimed in any of claims 1 to 3 where
R³ is hydrogen, halogen, cyano, C₁-C₆-alkyl, C₁-C₆-haloalkyl or C₁-C₆-alkoxy.

5. An N-heterocyclyl-substituted thienyloxypyrimidine of the formula I as claimed in any of claims 1 to 4 where
R⁴, R⁵, R⁶ are hydrogen, halogen, cyano, C₁-C₆-alkyl, C₁-C₆-haloalkyl or C₁-C₆-haloalkoxy.

6. A process for preparing N-heterocyclyl-substituted thienyloxypyrimidines of the formula I as claimed in claim 1, which comprises reacting pyrimidines of the formula II 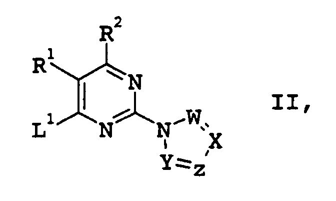 where W, X, Y, Z, R¹ and R² are as defined in claim 1 and L¹ is a nucleophilically displaceable leaving group with a thiophene derivative of the formula III 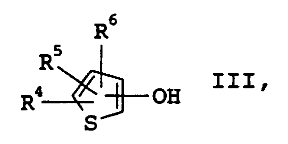 where R⁴, R⁵ and R⁶ are as defined in claim 1.

7. A process for preparing N-heterocyclyl-substituted thienyloxypyrimidines of the formula I as claimed in claim 1, which comprises reacting thienyloxypyrimidine derivatives of the formula IV 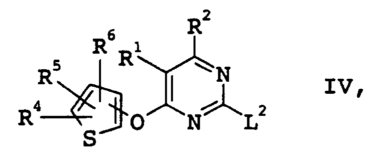 where R¹, R², R⁴, R⁵ and R⁶ are as defined in claim 1 and L² is a nucleophilically displaceable leaving group with a nitrogen heterocycle of the formula V 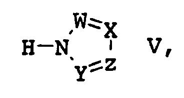 where W, X, Y and Z are as defined in claim 1.

8. A thienyloxypyrimidine derivative of the formula IV 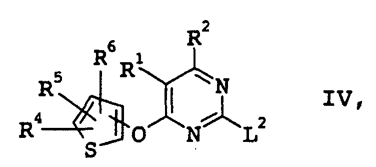 where R¹, R², R⁴, R⁵ and R⁶ are as defined in claim 1 and L² is a nucleophilically displaceable leaving group.

9. A composition, comprising a herbicidally effective amount of at least one N-heterocyclyl-substituted thienyloxypyrimidine of the formula I or an agriculturally useful salt of I as claimed in any of claims 1 to 5 and auxiliaries customary for formulating crop protection agents.

10. A process for preparing compositions as claimed in claim 9, which comprises mixing a herbicidally effective amount of at least one N-heterocyclyl-substituted thienyloxypyrimidine derivative of the formula I as claimed in any of claims 1 to 5 or an agriculturally useful salt of I and auxiliaries customary for formulating crop protection agents.

11. A method for controlling undesirable vegetation, which comprises allowing a herbicidally effective amount of at least one N-heterocyclyl-substituted thienyloxypyrimidine derivative of the formula I as claimed in any of claims 1 to 5 or an agriculturally useful salt of I to act on plants, their habitat and/or on seeds.

12. The use of the N-heterocyclyl-substituted thienyloxypyrimidine derivatives of the formula I as claimed in any of claims 1 to 5 and their agriculturally useful salts as herbicides.

## Revendications

1. Thiényloxypyrimidines N-hétérocyclylsubstituées de formule I 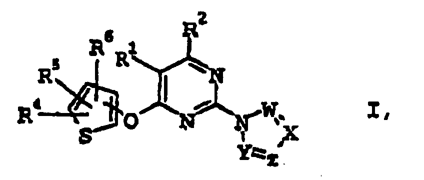 dans laquelle les variables ont les significations suivantes. :
W, X, Y, Z désignent, indépendamment l'une de l'autre, N ou CR³, au moins une des variables désignant CR³ ;
R¹ désigne l'hydrogène, un halogène, un radical cyano, alkyle C₁-C₆, halogénoalkyle C₁-C₆, alcoxy C₁-C₆ ou halogénoalcoxy C₁-C₆ ;
R² désigne l'hydrogène, un halogène, un radical cyano, alkyle C₁-C₆, alcényle C₂-C₆, alcynyle C₂-C₆, halogénoalkyle C₁-C₆, halogénoalcényle C₂-C₆, halogénoalcynyle C₂-C₆, alcoxy C₁-C₆, alcényloxy C₃-C₆, alcynyloxy C₃-C₆, halogénoalcoxy C₁-C₆, C₁-C₆-alcoxy-C₁-C₄-alkyle, alkylamino C₁-C₆, di-(C₁-C₄-alkyl)amino, alkylthio C₁-C₆, halogénoalkylthio C₁-C₆, alkylsulfinyle C₁-C₆, halogénoalkylsulfinyle C₁-C₆, alkylsulfonyle C₁-C₆, halogénoalkylsulfonyle C₁-C₆, COOR⁷ ou CONR⁸R⁹ ;
R³ désigne l'hydrogène, un halogène, un radical cyano, nitro, alkyle C₁-C₆, halogénoalkyle C₁-C₆, alcoxy C₁-C₆, halogénoalcoxy C₁-C₆, alkylthio C₁-C₆, halogénoalkylthio C₁-C₆, alkylsulfonyle C₁-C₆ ou COOR⁷ ;
R⁴, R⁵, R⁶ désignent l'hydrogène, un halogène, un radical cyano, alkyle C₁-C₆, halogénoalkyle C₁-C₆, alcoxy C₁-C₆, halogénoalcoxy C₁-C₆, alkylthio C₁-C₆, halogénoalkylthio C₁-C₆, alkylsulfonyle C₁-C₆ ou halogénoalkylsulfonyle C₁-C₆ ;
R⁷ désigne l'hydrogène, un radical alkyle C₁-C₄, alcényle C₃-C₄, alcynyle C₃-C₄ ou halogénoalkyle C₁-C₄ ;
R⁸ désigné l'hydrogène, un radical alkyle C₁-C₄, alcényle C₃-C₄, alcynyle C₃-C₄ ou alcoxy C₁-C₄ ;
R⁹ désigne l'hydrogène, un radical alkyle C₁-C₄, alcényle C₃-C₄ ou alcynyle C₃-C₄ ;
ainsi que leurs sels à usage agricole.

2. Thiényloxypyrimidines N-hétérocycylsubstituées de formule I selon la revendication 1, dans laquelle les variables
W, X, Y, Z désignent indépendamment l'une de l'autre N ou CR³, une des variables désignant N au maximum.

3. Thiényloxypyrimidines N-hétérocyclylsubstituées de formule I selon l'une des revendications 1 ou 2, dans laquelle
R¹ désigne l'hydrogène, un halogène, un radical cyano, alkyle C₁-C₆, halogénoalkyle C₁-C₆ ou alcoxy C₁-C₆;
R² désigne l'hydrogène, un halogène, un radical cyano, alkyle C₁-C₆, halogénoalkyle C₁-C₆, alcoxy C₁-C₆, halogénoalcoxy C₁-C₆, alkylthio C₁-C₆ ou halogénoalkylthio C₁-C₆.

4. Thiényloxypyrimidines N-hétérocyclylsubstituées de formule I selon l'une des revendications 1 à 3; dans laquelle
R³ désigne l'hydrogène, un halogène, un radical cyano, alkyle C₁-C₆, halogénoalkyle C₁-C₆ ou alcoxy C₁-C₆.

5. Thiényloxypyrimidines N-hétérocyclylsubstituées de formule I selon l'une des revendications 1 à 4, dans laquelle
R⁴, R⁵, R⁶ désignent l'hydrogène, un halogène, un radical cyano, alkyle C₁-C₆, halogénoalkyle C₁-C₆ ou halogénoalcoxy C₁-C₆.

6. Procédé de préparation de thiényloxypyrimidines N-hétérocyclylsubstituées de formule I selon la revendication 1, **caractérisé en ce que** l'on fait réagir des pyrimidines de formule II 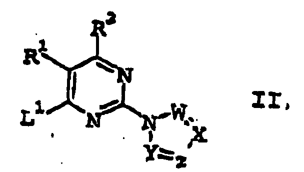 dans laquelle W, X, Y, Z, R¹ et R² ont les significations spécifiées dans la revendication 1 et L¹ désigne un groupe partant échangeable de manière nucléophile,
avec un dérivé de thiophène de formule III 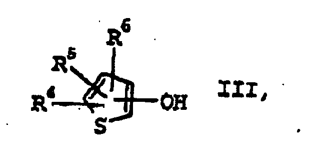 dans laquelle R⁴, R⁵ et R⁶ ont les significations spécifiées dans la revendication 1.

7. Procédé de préparation de thiényloxypyrimidines N-hétérocycylsubstituées de formule I selon la revendication 1, **caractérisé en ce que** l'on fait réagir des dérivés de thiényloxypyrimidines de formule IV 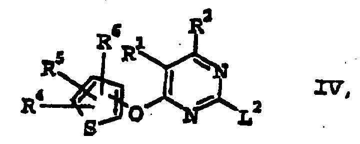 dans laquelle R¹, R², R⁴, R⁵ et R⁶ ont les significations spécifiées dans la revendication 1 et L² désigne un groupe partant échangeable de manière nucléophile,
avec un hétérocycle azoté de formule V 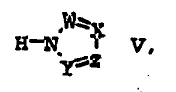 dans laquelle W, X, Y et Z ont les significations spécifiées dans la revendication 1.

8. Dérivés de thiényloxypyrimidine de formule IV 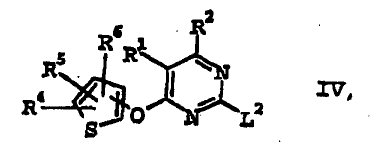 dans laquelle R¹, R², R⁴, R⁵ et R⁶ ont les significations spécifiées dans la revendication 1 et L² désigne un groupe partant échangeable de manière nucléophile.

9. Produit contenant une quantité active du point de vue herbicide d'au moins une thiényloxypyrimidine N-hétérocycylsubstituée de formule I ou d'un sel à usage agricole de I selon les revendications 1 à 5 et des adjuvants courants pour la formulation de produits phytoprotecteurs.

10. Procédé de préparation de produits selon la revendication 9, **caractérisé en ce que** l'on mélange une quantité active du point de vue herbicide d'au moins un dérivé de thiényloxypyrimidine N-hétérocyclylsubstituée de formule I selon les revendications 1 à 5 ou d'un sel à usage agricole de I et des adjuvants courants pour la formulation de produits phytoprotecteurs.

11. Procédé de lutte contre la croissance de plantes indésirées, **caractérisé en ce qu'**on fait agir une quantité active du point de vue herbicide d'au moins un dérivé de thiényloxypyrimidine N-hétérocyclylsubstituée de formule I selon les revendications 1 à 5 ou d'un sel à usage agricole de I sur des plantes, leur biotope et/ou des graines.

12. Utilisation des dérivés de thiényloxypyrimidine N-hétérocyclylsubstituées de formule I selon les revendications 1 à 5 et de leurs sels à usage agricole comme herbicides.
